# EUROPEAN PATENT APPLICATION

(11) **EP 4 748 930 A1**
(43) Date of publication of application: **27.05.2026**
(21) Application number: 24859644.7
(22) Date of filing: 23.08.2024
(51) Int. Cl.: C12N 9/22, C12N 9/16, C12N 15/09, C12N 15/11, C12N 15/55

(54) **ENGINEERED PROTEIN**

(30) Priority: 25.08.2023 JP 2023137565; 15.04.2024 JP 2024065568
(71) Applicant: The University of Tokyo, Bunkyo-ku, Tokyo 113-8654 (JP); Kyoto Prefectural Public University Corporation, Kyoto 602-8566 (JP); Jichi Medical University, Tokyo 102-0093 (JP); National Agriculture and Food Research Organization, Tsukuba-shi, Ibaraki 305-8517 (JP)
(72) Inventor: NUREKI, Osamu, Tokyo 113-8654 (JP); NAKAGAWA, Ryoya, Tokyo 113-8654 (JP); OMURA, Satoshi, Tokyo 113-8654 (JP); KISE, Yoshiaki, Tokyo 160-0022 (JP); HOSHINO, Atsushi, Kyoto-shi, Kyoto 602-8566 (JP); HINO, Tomohiro, Kyoto-shi, Kyoto 602-8566 (JP); MATOBA, Satoaki, Kyoto-shi, Kyoto 602-8566 (JP); OHMORI, Tsukasa, Shimotsuke-shi, Tochigi 329-0498 (JP); SAIKA, Hiroaki, Tsukuba-shi, Ibaraki 305-8634 (JP); TOKI, Seiichi, Tsukuba-shi, Ibaraki 305-8634 (JP); ISHIBASHI, Kazuhiro, Tsukuba-shi, Ibaraki 305-8634 (JP)
(74) Representative: Manitz Finsterwald Patent- und Rechtsanwaltspartnerschaft mbB
(86) International application number: PCT/JP2024/030010
(87) International publication number: WO 2025/047611

(57) **Abstract**

The present invention provides an engineered protein that can be utilized as a genome engineering tool. The protein includes a substitution to histidine at amino acid position 188 in an amino acid sequence as set forth in SEQ ID NO: 1, and further includes one substitution selected from: a substitution to tyrosine at amino acid position 2; a substitution to tyrosine at amino acid position 70; a substitution to arginine at amino acid position 80; a substitution to threonine at amino acid position 105; a substitution to histidine at amino acid position 123; a substitution to lysine at amino acid position 195; a substitution to arginine at amino acid position 208; a substitution to alanine at amino acid position 232; a substitution to methionine at amino acid position 246; a substitution to methionine at amino acid position 316; and a substitution to isoleucine at amino acid position 337.

## Description

### Technical Field

The present invention relates to an engineered substance, and for example, relates to an engineered protein.

### Background Art

Bacterial and archaeal CRISPR-Cas systems have each been known to provide adaptive immunity against a foreign nucleic acid. In Patent Literature 1, there is a disclosure that the CRISPR-Cas systems are classified into two classes (Classes 1 and 2) and six types (Types I to VI). The Class 2 system includes Types II, V, and VI, and includes a single multi-domain effector Cas protein, such as Cas9 (Type II) or Cas12 (Type V).

As disclosed in Patent Literature 1, Cas9 and Cas12a have been widely used as versatile genome engineering tools because the proteins each exhibit strong nuclease activity in a eukaryotic cell.

### Citation List

### Patent Literature

[PTL 1] WO 2022/092317 A1

### Summary of Invention

### Technical Problem

Proteins used as genome engineering tools, such as Cas9 and Cas12, are required to have various characteristics.

For example, SpCas9 derived from *Streptococcus pyogenes* is suitable for a genome engineering tool because SpCas9 has high activity. However, its molecular size is as large as 1,367 amino acid residues (the length of its gene is 4.1 kb). Meanwhile, in an AAV vector, space for a foreign gene is less than 4.5 kb, and hence even the following is difficult: SpCas9 is loaded onto the AAV vector and introduced into an animal cell. Accordingly, SpCas9 is limited by its size when used as a genome engineering tool.

Accordingly, it has been required to provide an engineered protein that can be utilized as a genome engineering tool.

The present invention has been made under the above-mentioned background, and an object of the present invention is to provide an engineered substance that can be utilized as a genome engineering tool.

### Solution to Problem

According to one aspect of the present invention, there is provided a protein, including a substitution to histidine at amino acid position 188 in an amino acid sequence as set forth in SEQ ID NO: 1, wherein the protein further includes one substitution selected from: a substitution to tyrosine at amino acid position 2; a substitution to tyrosine at amino acid position 70; a substitution to arginine at amino acid position 80; a substitution to threonine at amino acid position 105; a substitution to histidine at amino acid position 123; a substitution to lysine at amino acid position 195; a substitution to arginine at amino acid position 208; a substitution to alanine at amino acid position 232; a substitution to methionine at amino acid position 246; a substitution to methionine at amino acid position 316; and a substitution to isoleucine at amino acid position 337, and wherein the protein has a sequence identity of 90% or more with the amino acid sequence as set forth in SEQ ID NO: 1, and has cleavage activity against a target site of DNA.

### Advantageous Effects of Invention

According to the present invention, there can be provided the engineered substance that can be utilized as a genome engineering tool.

### Brief Description of Drawings

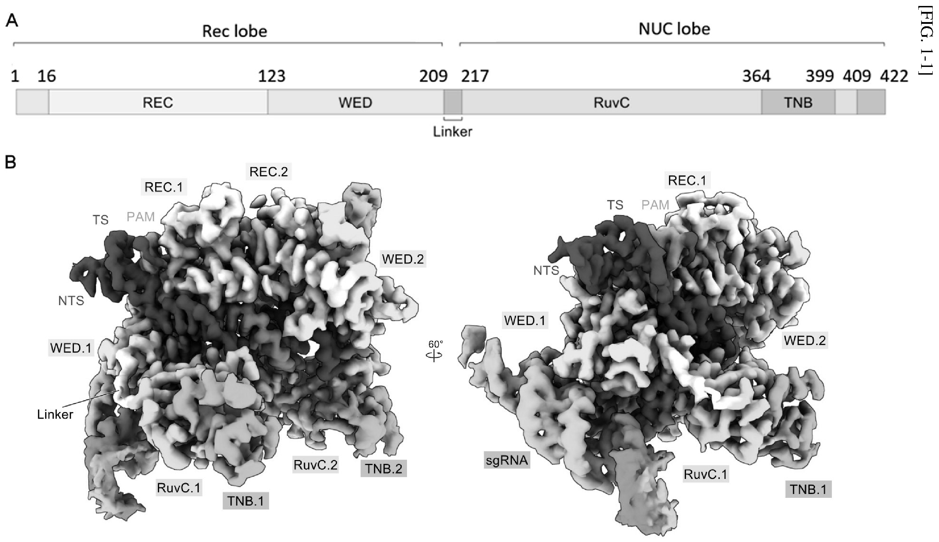

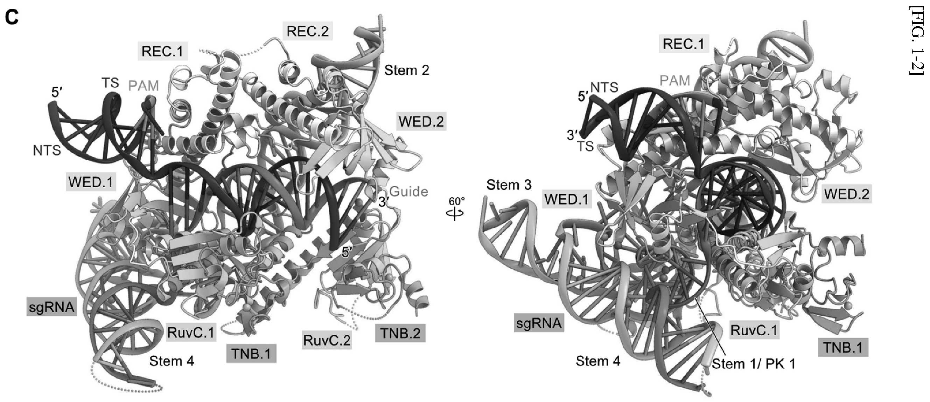

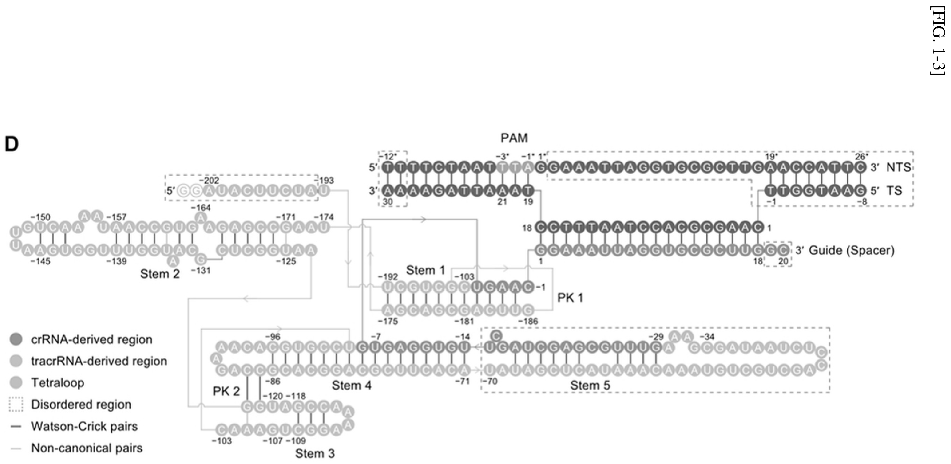

[FIG. 1-1] Part A is a view for illustrating the domain structure of AsCas12f, and Part B is a view for illustrating complex structure analysis with a cryo-electron microscope.
[FIG. 1-2] Part C is an illustration of the structural model of an AsCas12f-sgRNA-target DNA ternary complex.
[FIG. 1-3] Part D is a schematic view of sgRNA and target DNA.
[FIG. 2-1] Part A is a profile of size-exclusion chromatography, and Part B is an explanatory view of a typical cryo-electron microscopy image recorded on Titan Krios.
[FIG. 2-2] Part C is an explanatory view of a single-particle cryo-electron microscopy image processing workflow, and Part D is a graph showing a Fourier shell correlation (FSC) curve for 3D reconstruction.

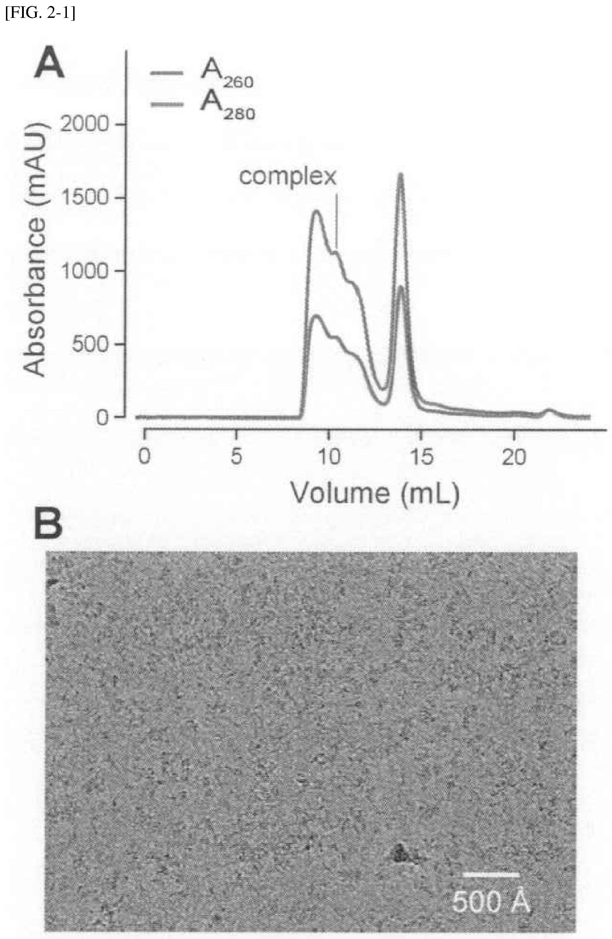

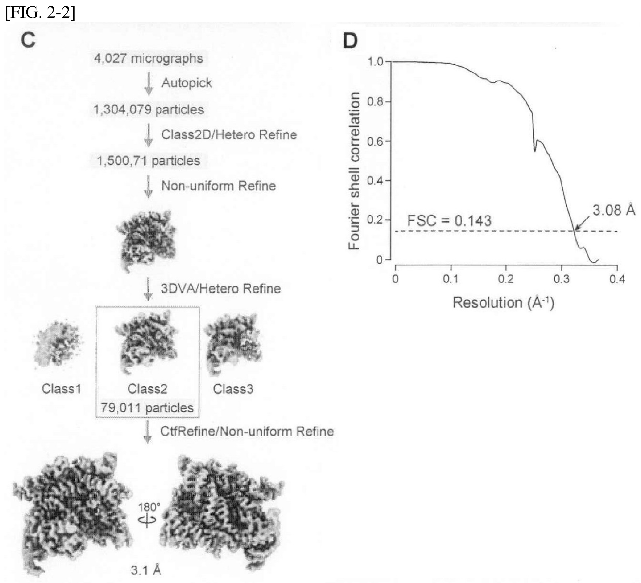

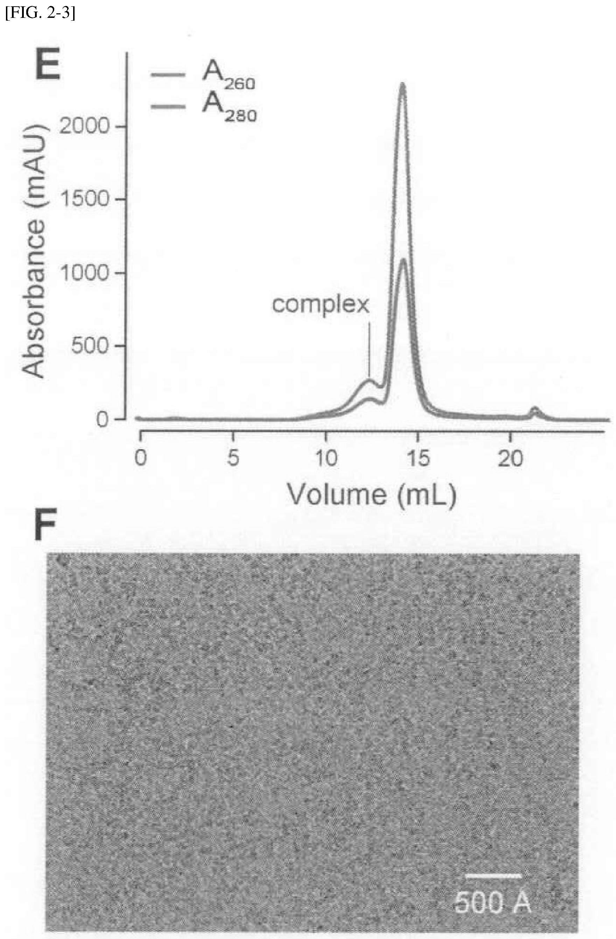

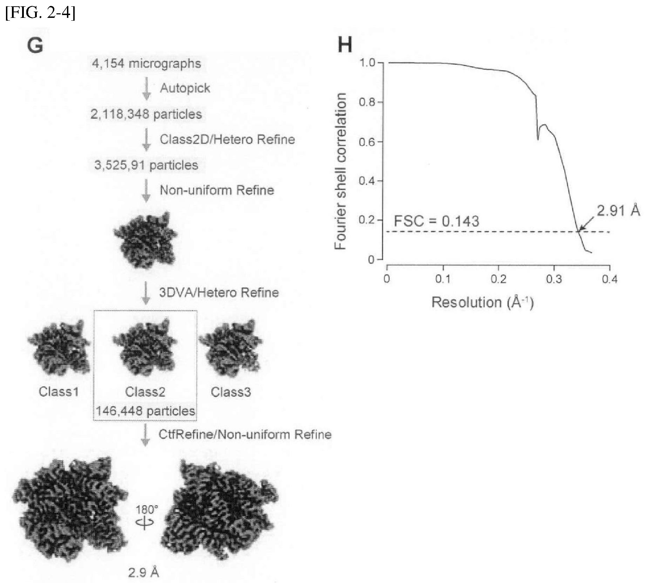

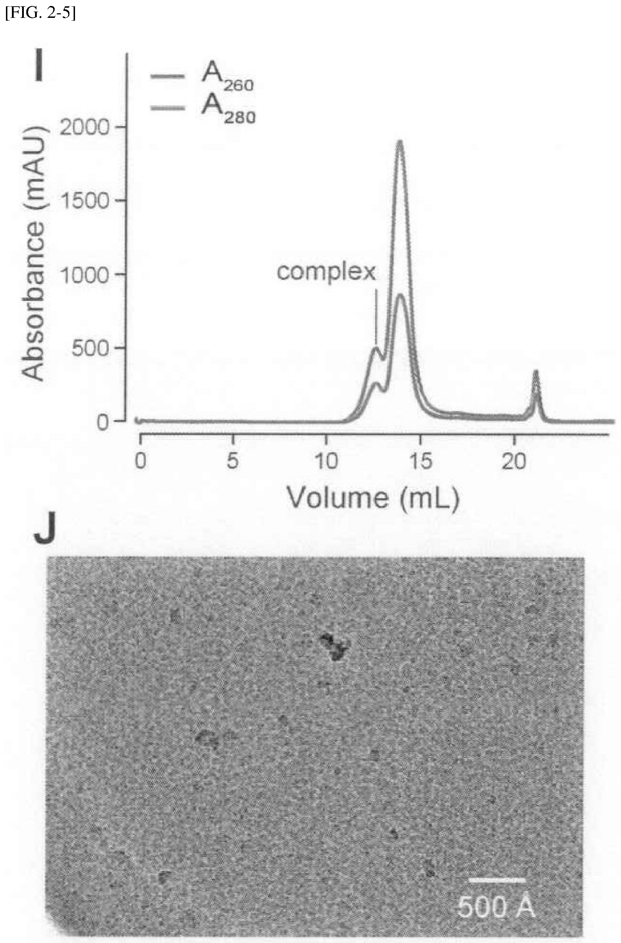

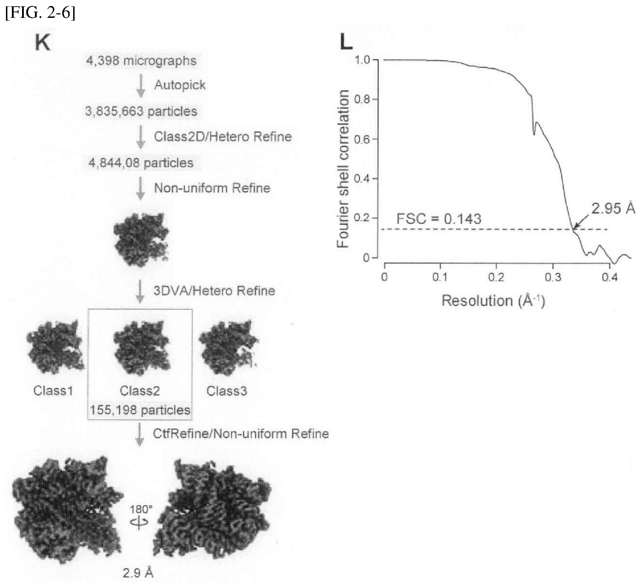

[FIG. 2-3] Part E is a profile of size-exclusion chromatography, and Part F is an explanatory view of a typical cryo-electron microscopy image recorded on the Titan Krios.
[FIG. 2-4] Part G is an explanatory view of a single-particle cryo-electron microscopy image processing workflow, and Part H is a graph showing a Fourier shell correlation (FSC) curve for 3D reconstruction.
[FIG. 2-5] Part I is a profile of size-exclusion chromatography, and Part J is an explanatory view of a typical cryo-electron microscopy image recorded on the Titan Krios.
[FIG. 2-6] Part K is an explanatory view of a single-particle cryo-electron microscopy image processing workflow, and Part L is a graph showing a Fourier shell correlation (FSC) curve for 3D reconstruction.

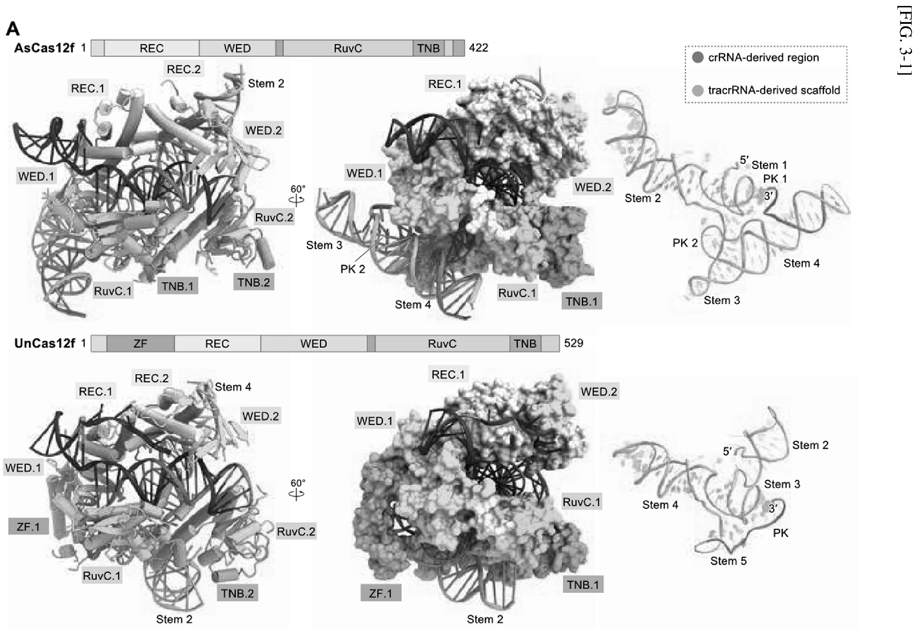

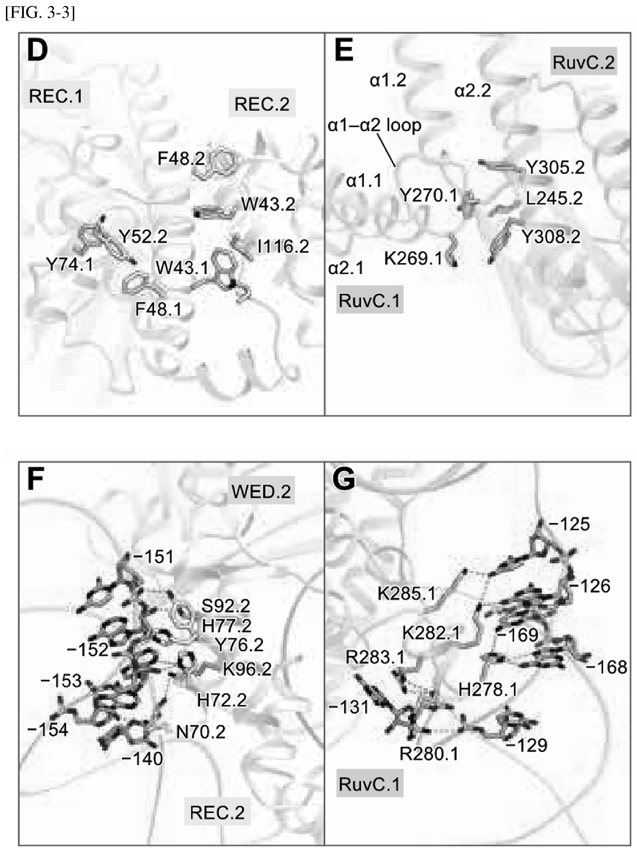

[FIG. 3-1] Part A is an explanatory view of structural comparison between AsCas12f and UnCas12f.

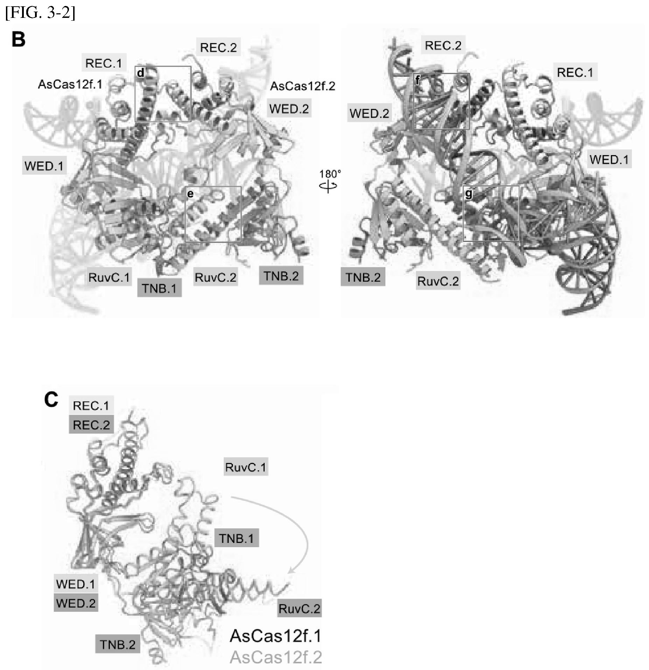

[FIG. 3-2] Part B is an explanatory view of a dimer interface between AsCas12f.1 and AsCas12f.2, and recognition sites for a sgRNA scaffold, and Part C is an explanatory view of the superposition of AsCas12f.1 and AsCas12f.2 based on a REC lobe.
[FIG. 3-3] Part D is an explanatory view of a dimer interface between REC.1 and REC.2, Part E is an explanatory view of a dimer interface between RuvC.1 and RuvC.2, Part F is an explanatory view of the recognition of Stem 2 by AsCas12f.2, and Part G is an explanatory view of the recognition of Stem 2 by AsCas12f.1.

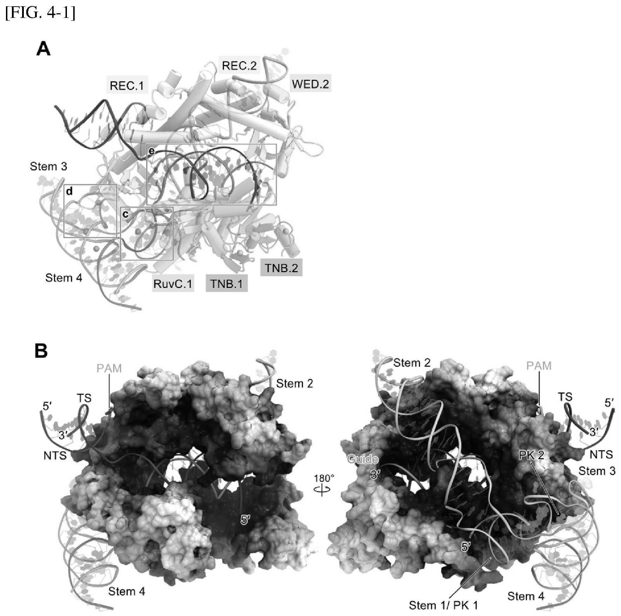

[FIG. 4-1] Part A is an explanatory view of recognition sites for guide RNA and target DNA, and Part B is an explanatory view of the electrostatic surface potential of AsCas12f.

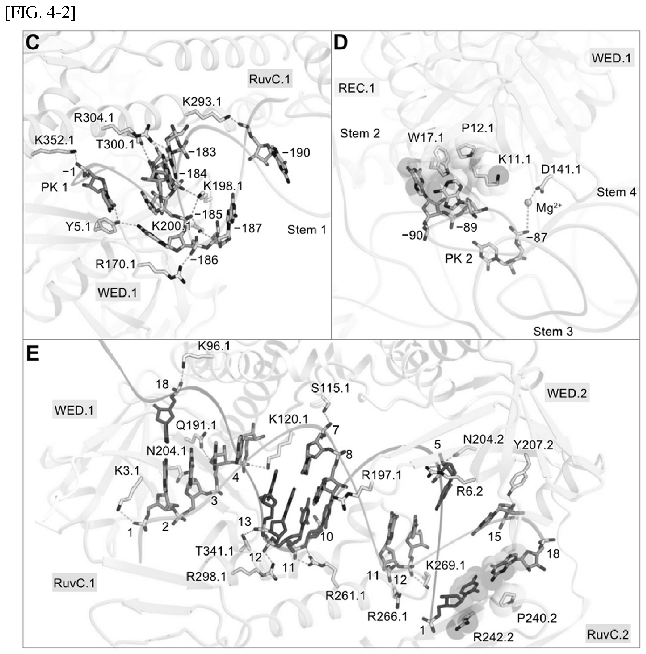

[FIG. 4-2] Parts C to E are explanatory views of the recognition of Stem 1 and PK1, Stem 3, and a guide RNA-target DNA heteroduplex, respectively.

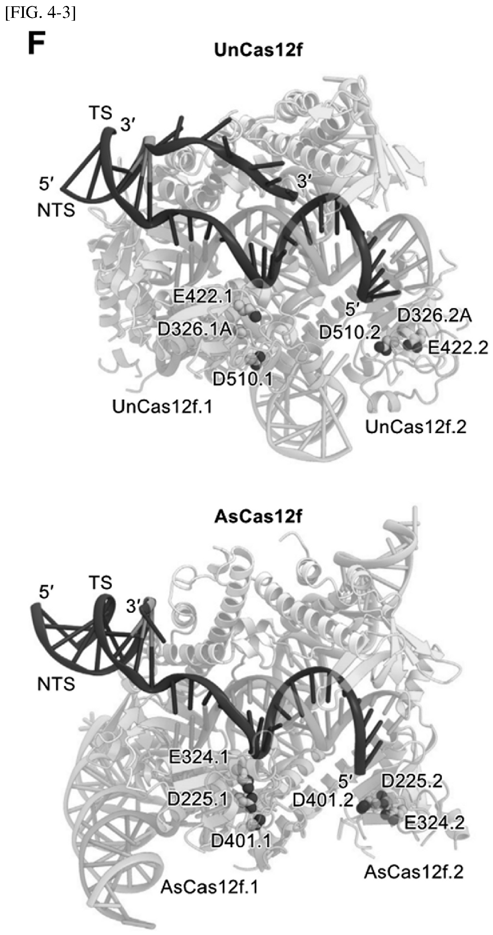

[FIG. 4-3] Part F is an explanatory view of the structural comparison of the TS, NTS, and RuvC active sites of AsCas12f with those of UnCas12f derived from an uncultured archaeon.

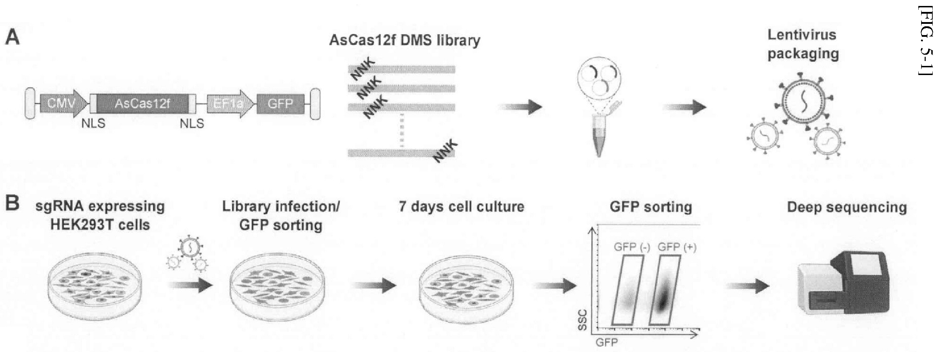

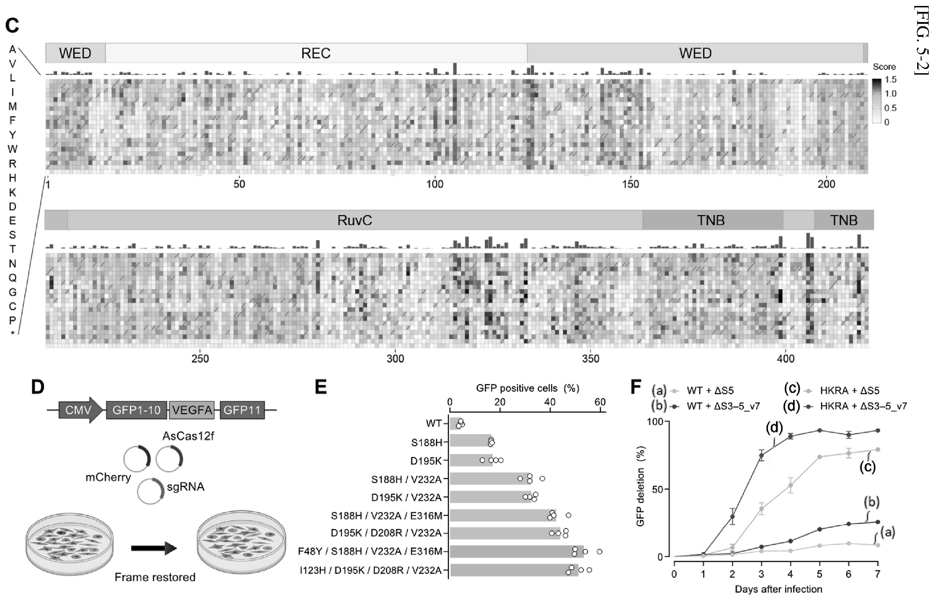

[FIG. 5-1] Part A is an explanatory view of a DMS library design for AsCas12f, and Part B is an explanatory view of the overview of a DMS approach for the genomic evaluation of gene editing efficiency in the context of GFP gene deletion.
[FIG. 5-2] Part C is an explanatory graph of the influence of a single mutation on genome editing activity, Part D is an explanatory view of a Split-GFP-reconstitution assay for evaluating genome editing activity against a VEGF gene in HEK293T cells, Part E is an explanatory graph of the genome editing activity of AsCas12f mutations, and Part F is a graph showing the temporal course of GFP deletion induced by AsCas12f-HKRA and AsCas12f in HEK293T cells each expressing d2EGFP.

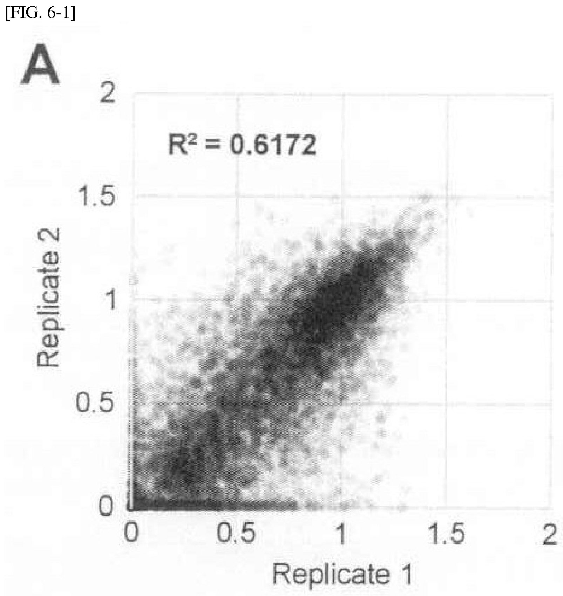

[FIG. 6-1] Part A is a graph showing a correlation between the effects of single variants on GFP deletion.

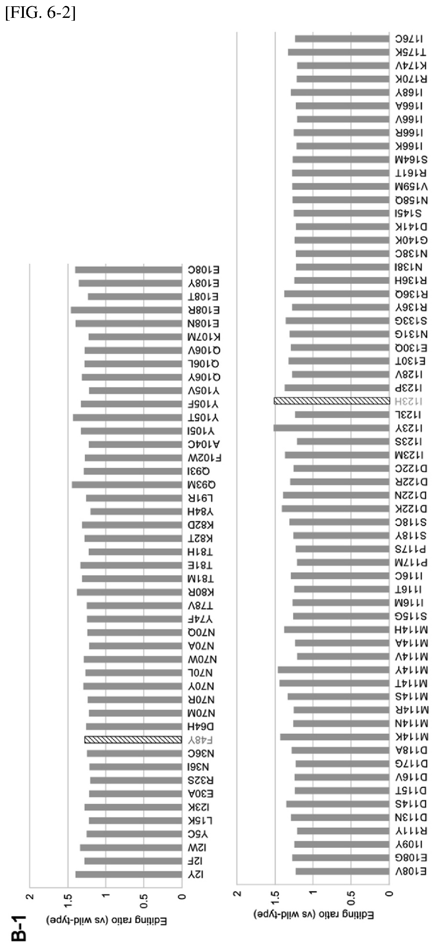

[FIG. 6-2] Part B-1 is a list of single amino acid substitutions in each of which editing efficiency increases by 20% or more as compared to that of a WT.

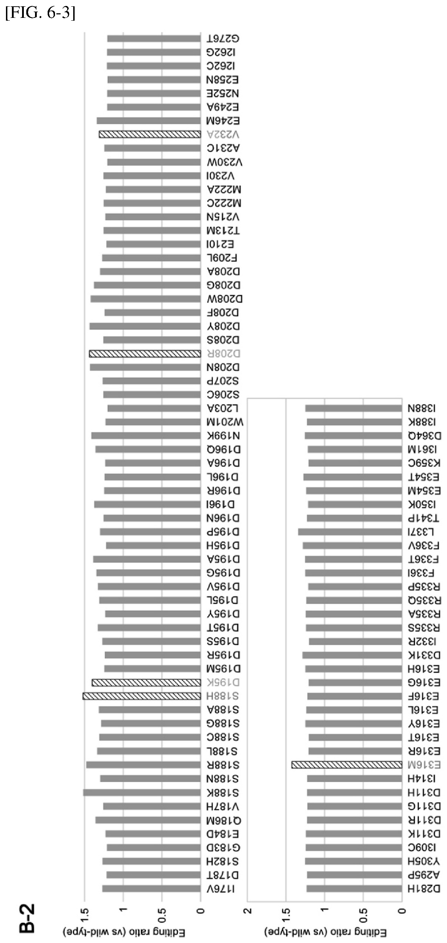

[FIG. 6-3] Part B-2 is a list of single amino acid substitutions in each of which editing efficiency increases by 20% or more as compared to that of the WT.

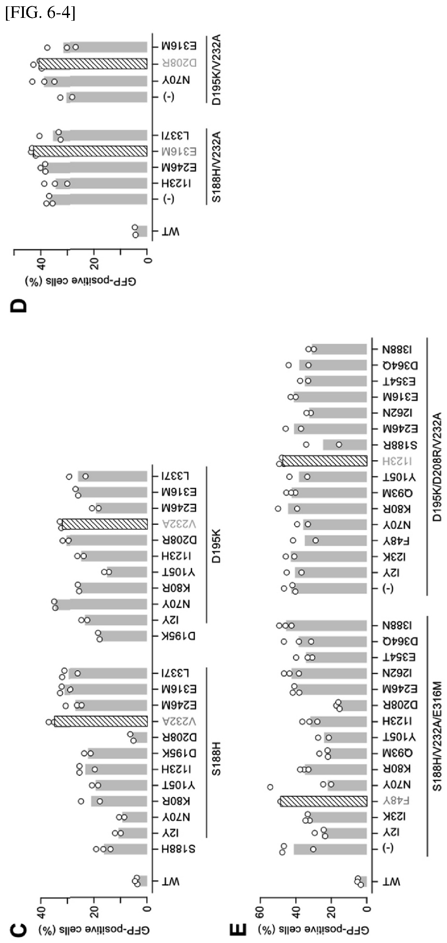

[FIG. 6-4] Parts C, D, and E are graphs showing the genome editing activities of double, triple, and quadruple AsCas12f variants, respectively.

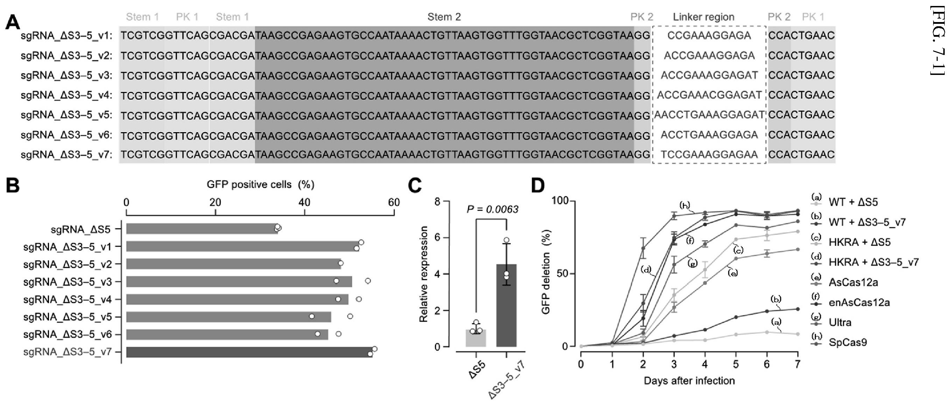

[FIG. 7-1] Part A is an explanatory view of the sequences of sgRNA variants, Part B is an explanatory graph of the genome editing activity of each of the sgRNA variants determined by flow cytometry, Part C is a graph showing the expression of sgRNA quantified by a qPCR for a common sequence site (n=3, mean±SD), and Part D is a graph showing the temporal course of GFP deletion.

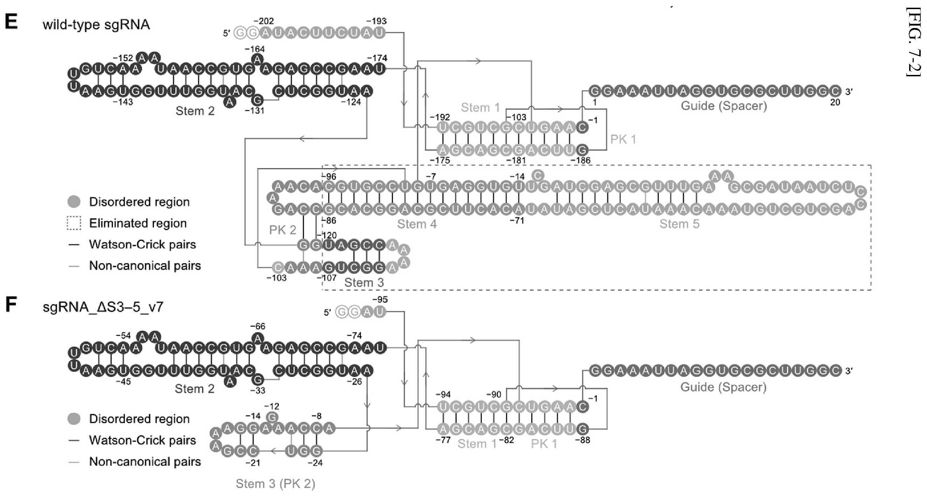

[FIG. 7-2] Part E is a schematic explanatory view of wild-type sgRNA, and Part F is a schematic explanatory view of sgRNA_ΔS3-5_v7.

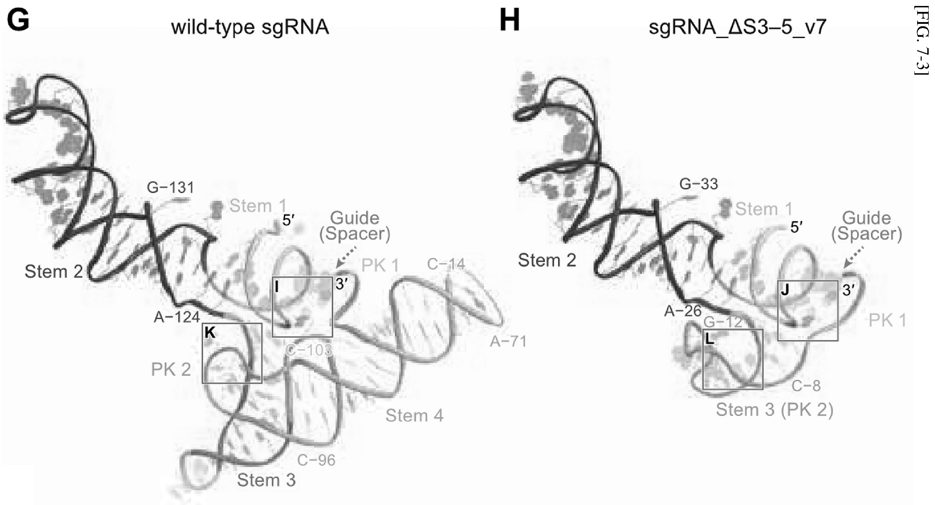

[FIG. 7-3] Part G is an explanatory view of the structure of a wild-type sgRNA scaffold, and Part H is an explanatory view of the structure of a sgRNA_ΔS3-5_v7 scaffold.

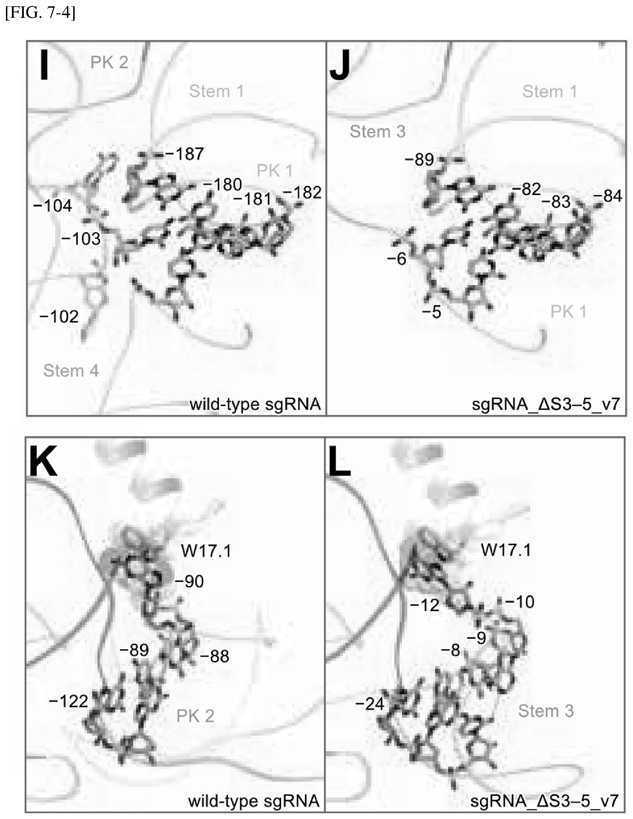

[FIG. 7-4] Part I is an enlarged view of the PK1 region of the wild-type sgRNA, Part J is an enlarged view of the PK1 region of sgRNA_ΔS3-5_v7, Part K is an enlarged view of the PK2 region of the wild-type sgRNA, and Part L is an enlarged view of the Stem 3 region of sgRNA_ΔS3-5_v7.

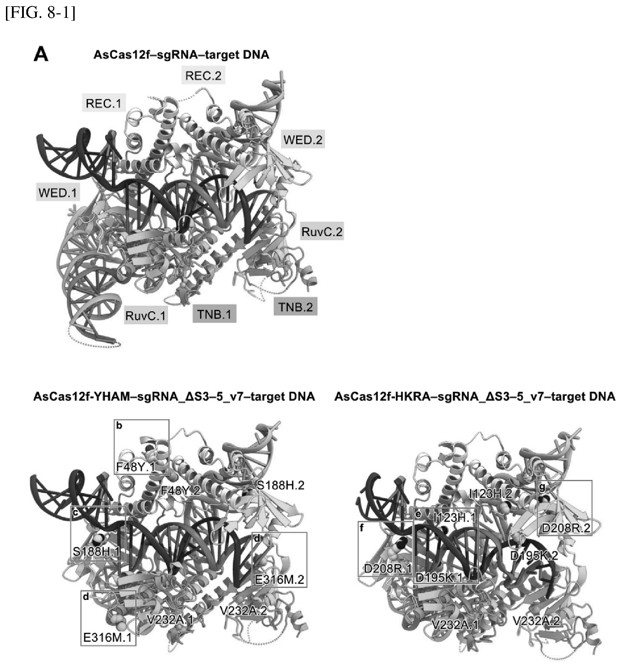

[FIG. 8-1] Part A is an explanatory view of results for target DNA and a target DNA complex with a cryo-electron microscope.

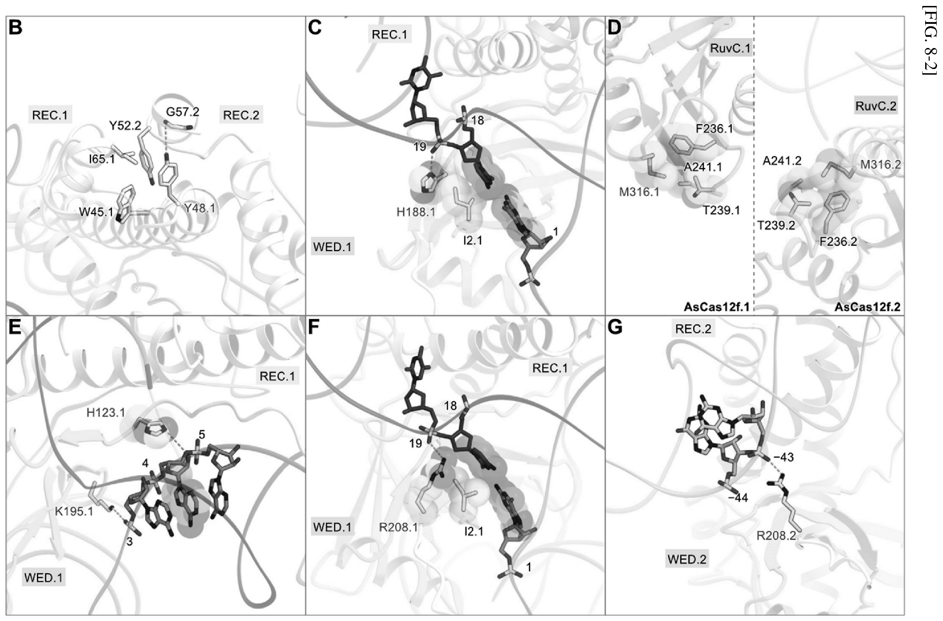

[FIG. 8-2] Part B is an explanatory view of the dimer interface of AsCas12f-YHAM, Part C is an explanatory view of the target DNA recognition of AsCas12f-YHAM, Part D is an explanatory view of the hydrophobic interaction of AsCas12f-YHAM, Part E is an explanatory view of the recognition of the guide RNA-target DNA heteroduplex of AsCas12f-HKRA, Part F is an explanatory view of the recognition of target DNA, and Part G is an explanatory view of the recognition of a sgRNA scaffold.

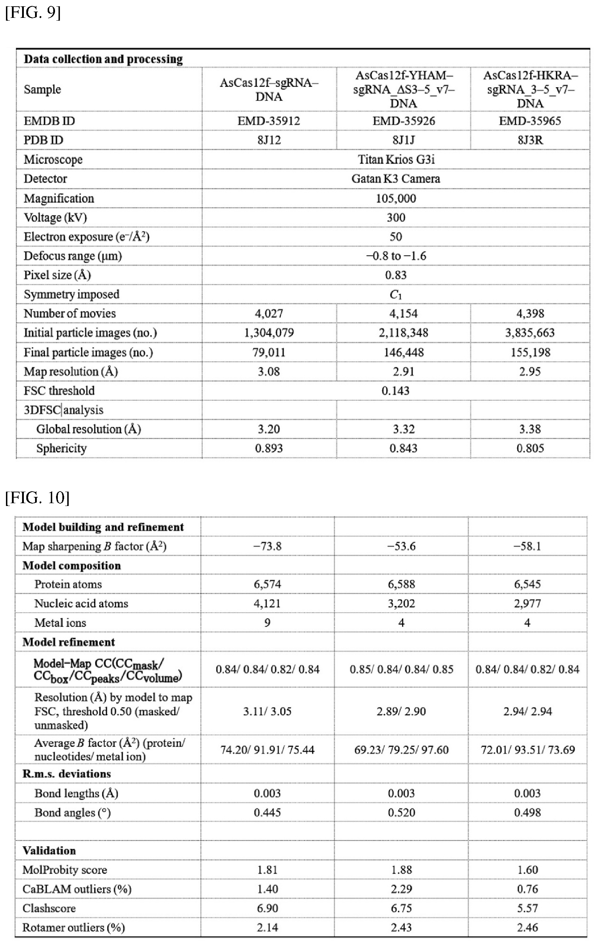

[FIG. 9] An explanatory table showing the results of data collection, processing, model refinement, and validation.
[FIG. 10] An explanatory table showing the results of data collection, processing, model refinement, and validation.

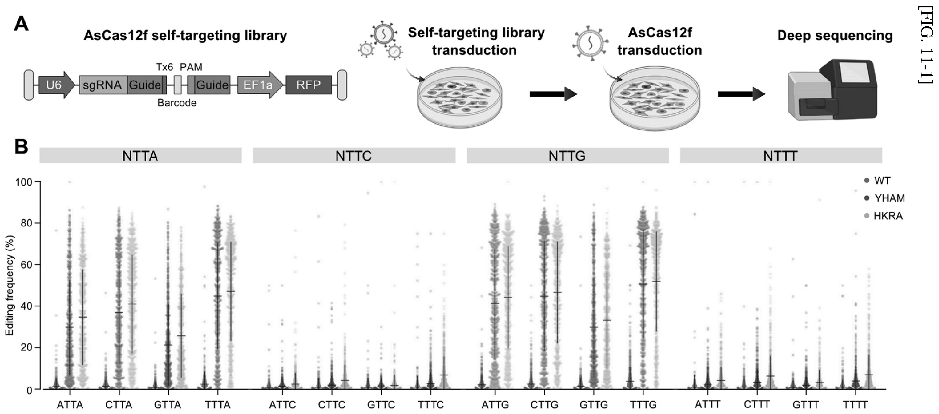

[FIG. 11-1] Part A is an explanatory view of a self-targeting library, and Part B is a graph showing an editing frequency.

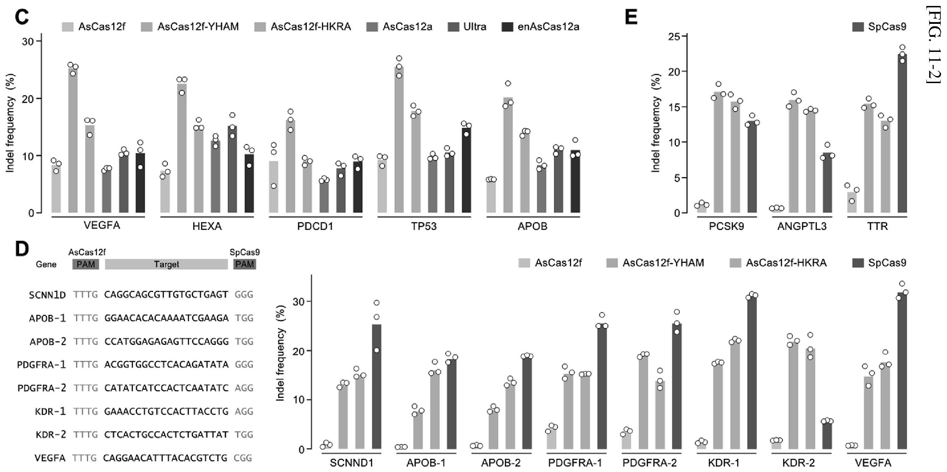

[FIG. 11-2] Parts C to E are graphs showing genome editing activities with various Cas enzymes.

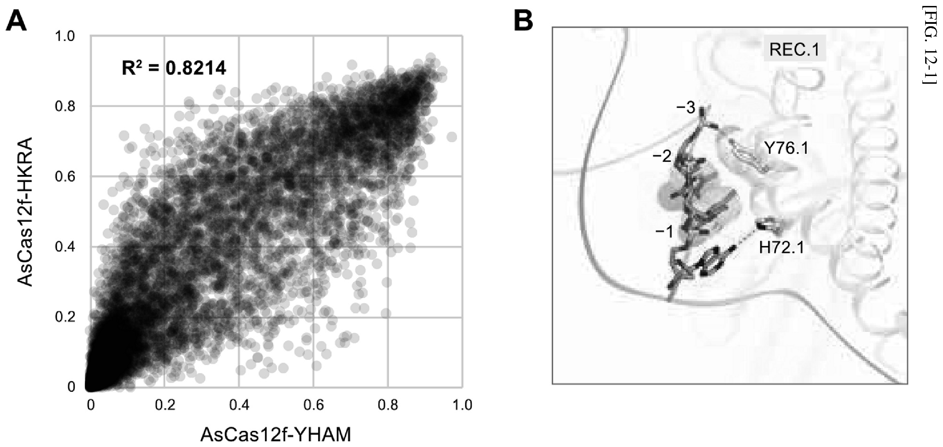

[FIG. 12-1] Part A is a graph showing the comparison results of target editing efficiency, and Part B is an explanatory view of the recognition results of a PAM duplex.

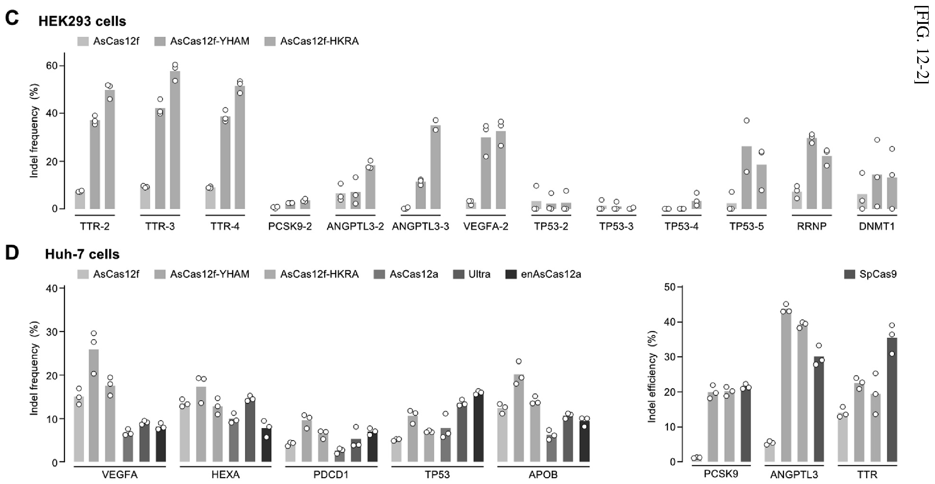

[FIG. 12-2] Parts C and D are each a graph showing indel efficiency at any other target site or the like.

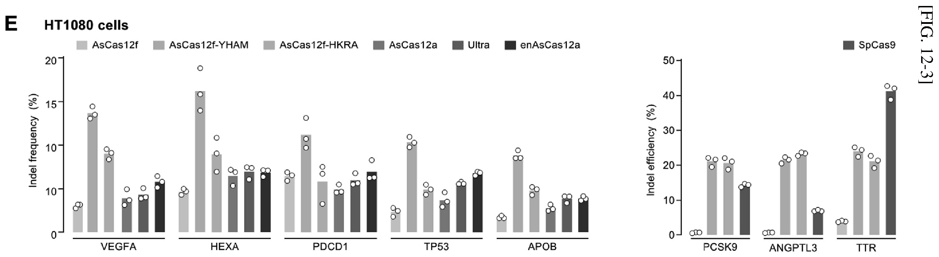

[FIG. 12-3] Part E is a graph showing indel efficiency at any other target site or the like.

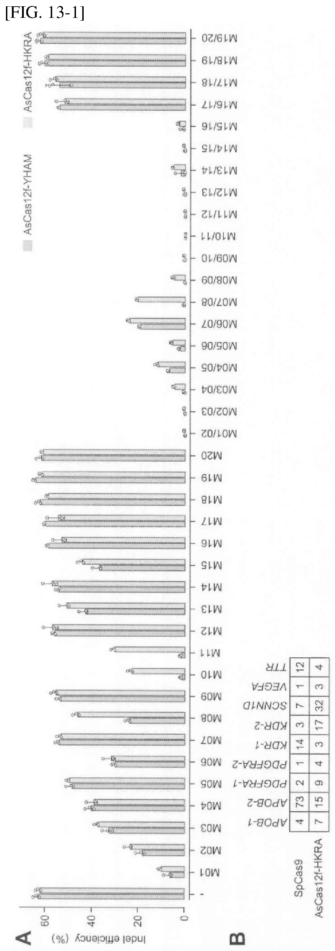

[FIG. 13-1] Part A is a graph showing the influence of a mismatch, and Part B is an explanatory view of comparison between the numbers of off-target edits.

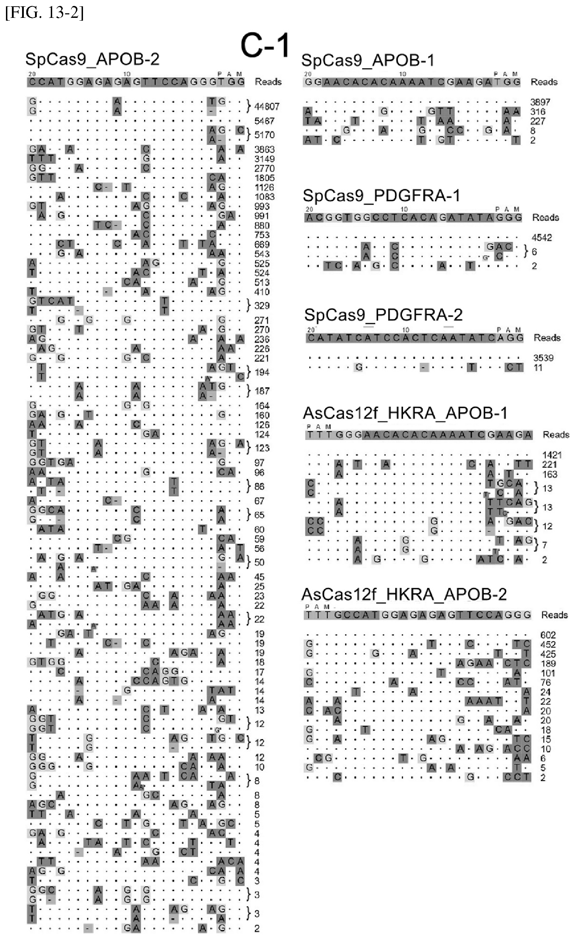

[FIG. 13-2] Part C-1 is an explanatory view of off-target sites identified by GUIDE-seq.

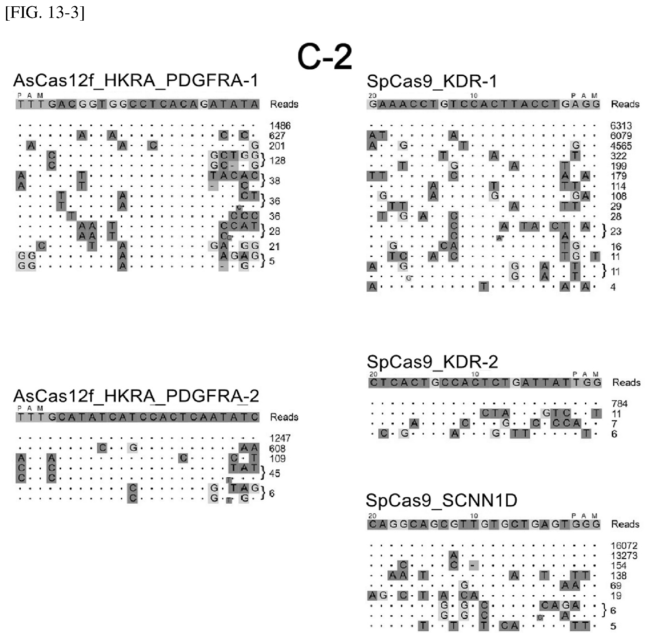

[FIG. 13-3] Part C-2 is an explanatory view of off-target sites identified by the GUIDE-seq.

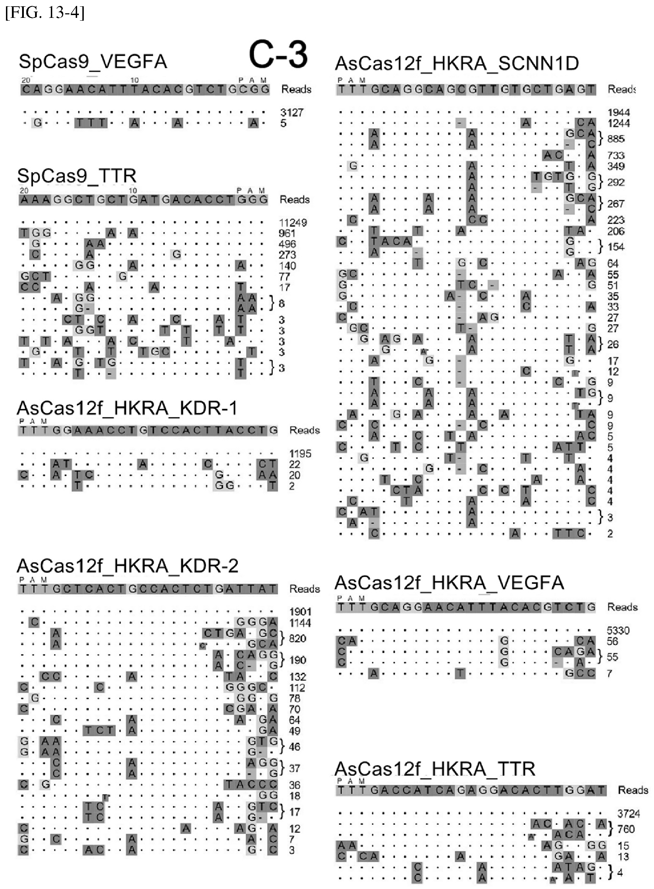

[FIG. 13-4] Part C-3 is an explanatory view of off-target sites identified by the GUIDE-seq.

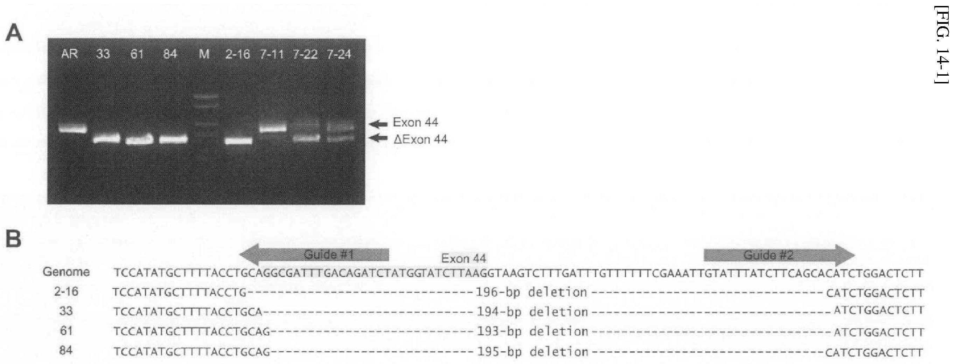

[FIG. 14-1] Explanatory views of the production of muscular dystrophy model induced pluripotent stem cells (iPSCs) and their differentiation into cardiomyocytes (iPSC-CMs), in which Part A is an explanatory view of the recognition of the complete deletion of a DMD exon by a PCR, and Part B is an explanatory view of the genomic sequence of a DMD exon 44-deleted iPS cell line.

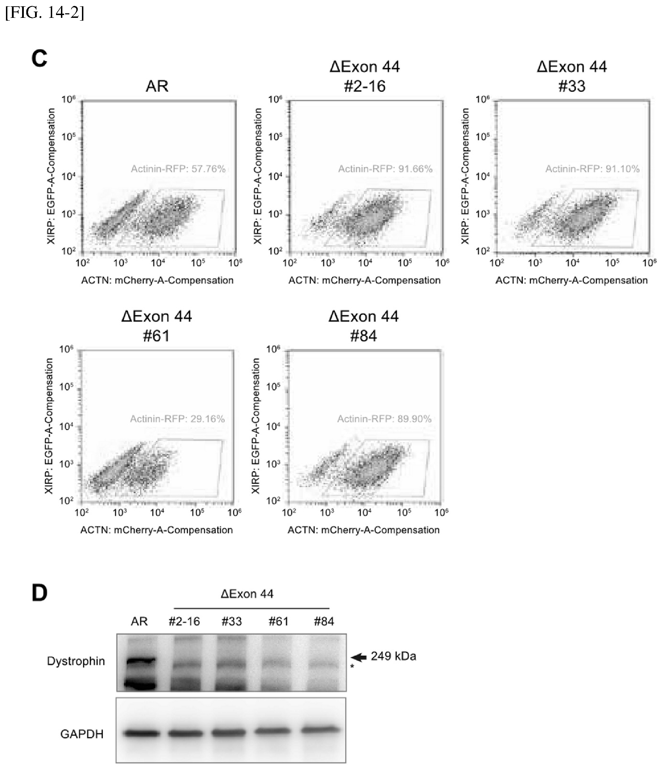

[FIG. 14-2] Explanatory views of the production of the muscular dystrophy model induced pluripotent stem cells (iPSCs) and their differentiation into the cardiomyocytes (iPSC-CMs), in which Part C is an explanatory view of the flow cytometry analysis of α-actinin-mCherry on day 14 of myocardial differentiation, and Part D is a view for illustrating Western blot analysis.

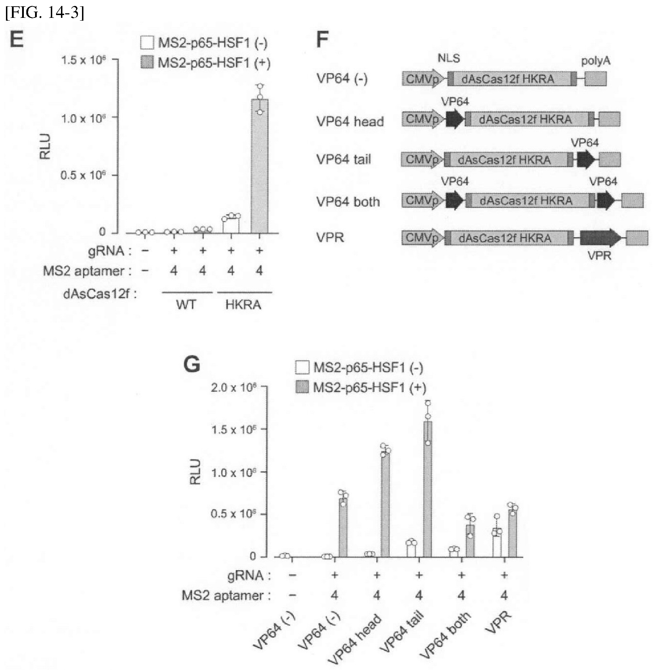

[FIG. 14-3] Part E is a graph showing luciferase activity comparison, Part F is a schematic explanatory view of a plasmid construct, and Part G is a graph showing an increase in luciferase activity in cells having introduced thereinto a plasmid vector.

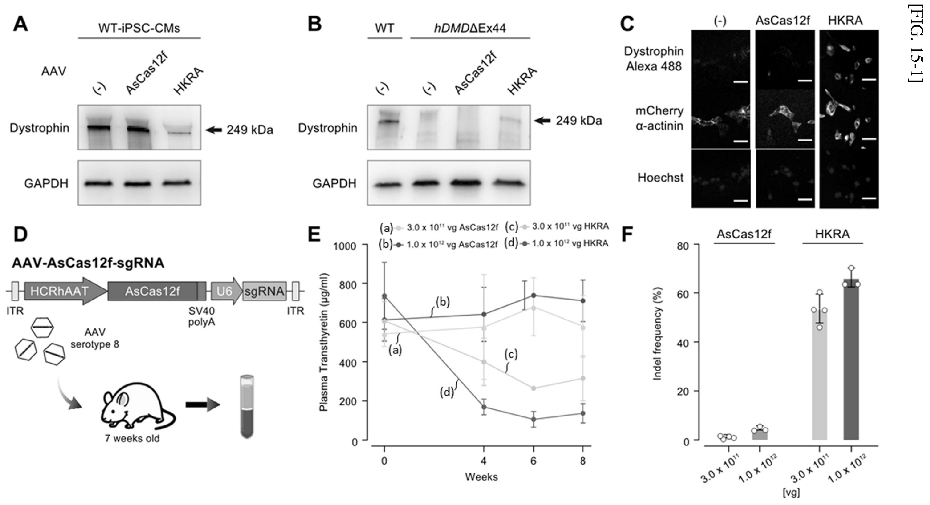

[FIG. 15-1] Parts A to F are explanatory views and graphs of treatment and knock-in with variants.

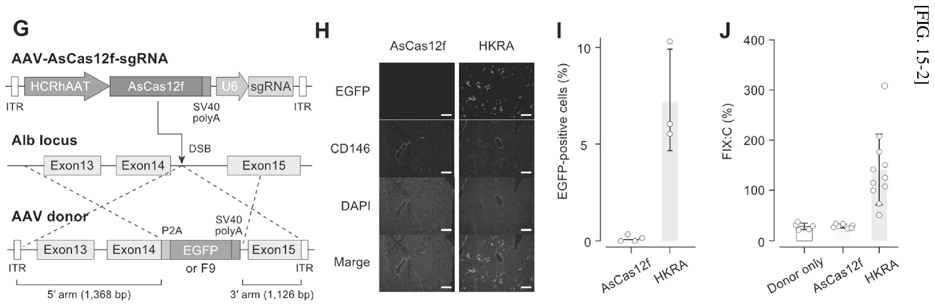

[FIG. 15-2] Parts G to J are explanatory views and graphs of the treatment and the knock-in with the variants.

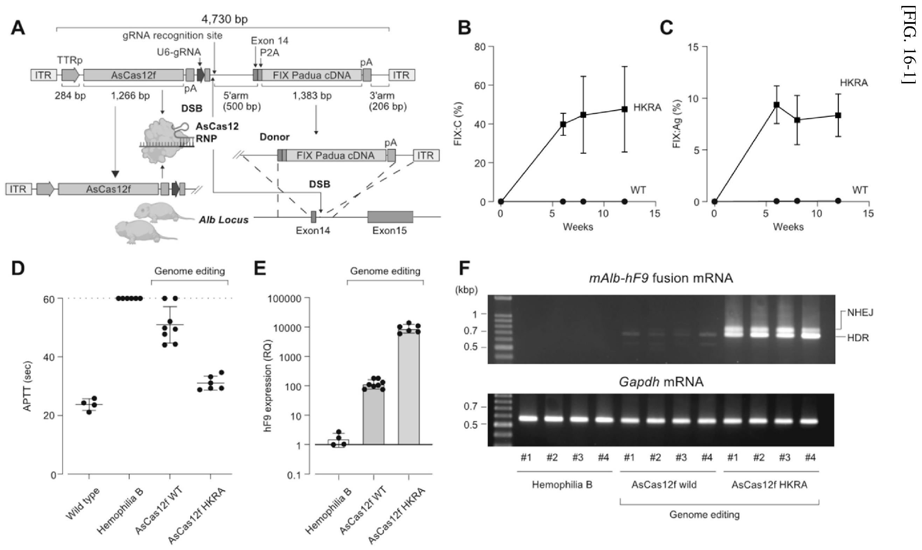

[FIG. 16-1] Parts A to F are explanatory views and graphs of the knock-in of FIX at an Alb locus (mice) with AsCas12f variants.

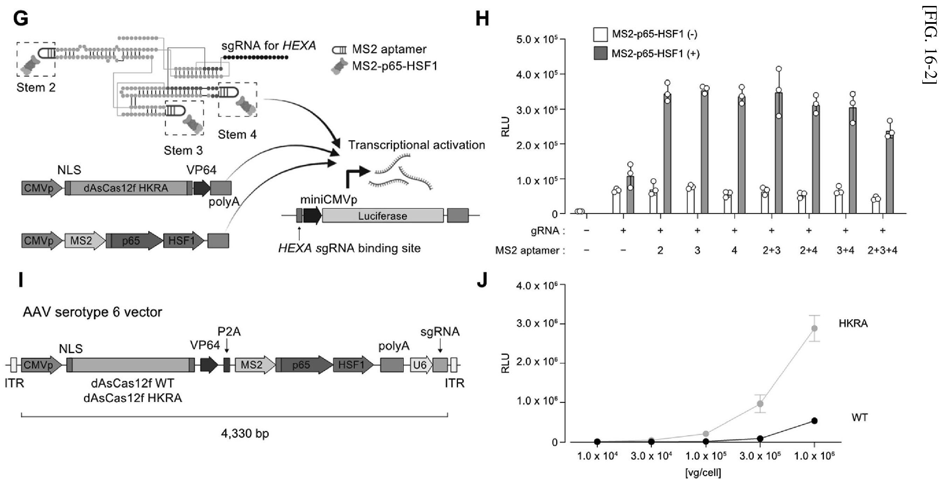

[FIG. 16-2] Parts G to J are explanatory views and graphs of gene transcriptional activation with the AsCas12f variants.

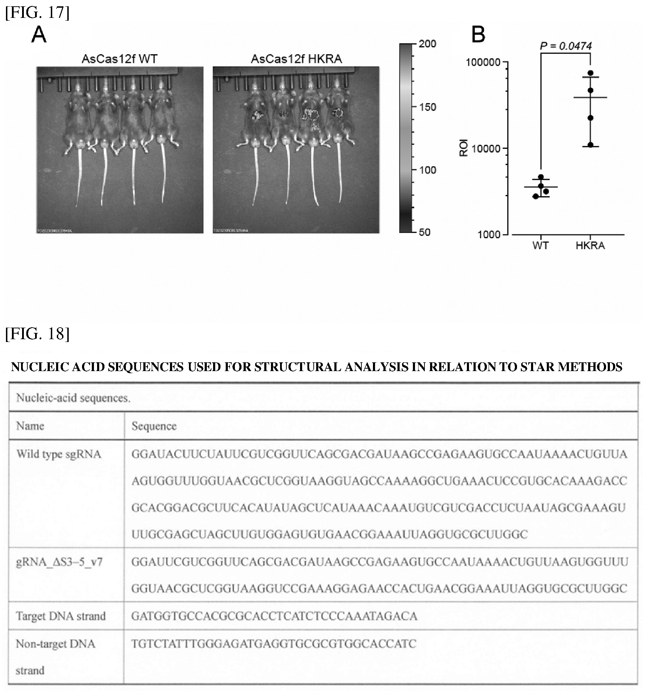

[FIG. 17] Part A is a pair of explanatory photographs showing the expression of a luciferase by gene transcriptional activation in mice, and Part B is a graph showing ROI values.
[FIG. 18] An explanatory table of nucleic acid sequences used for structural analysis in relation to STAR Methods.

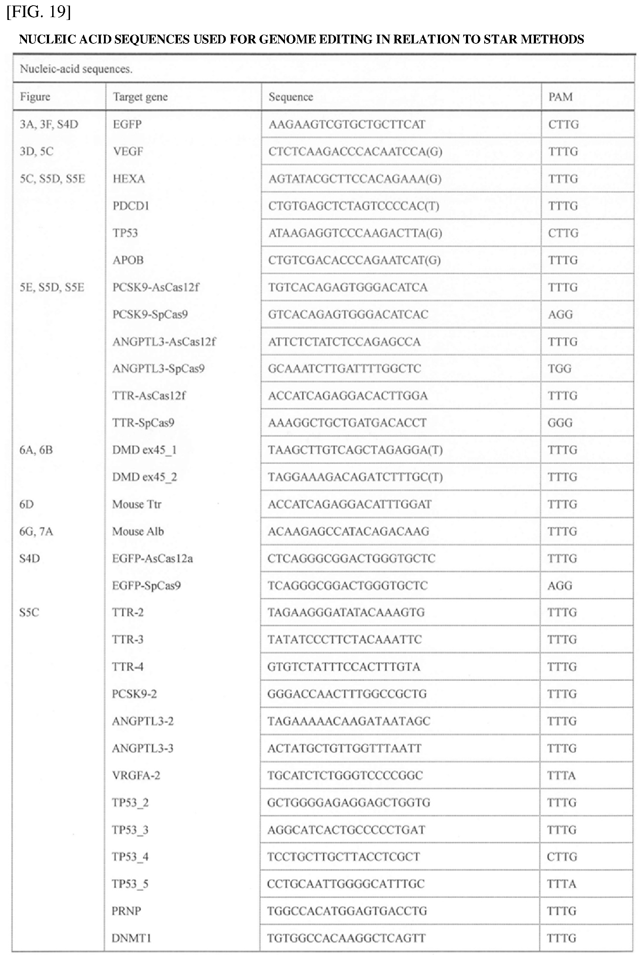

[FIG. 19] An explanatory table of nucleic acid sequences used for genome editing in relation to STAR Methods.

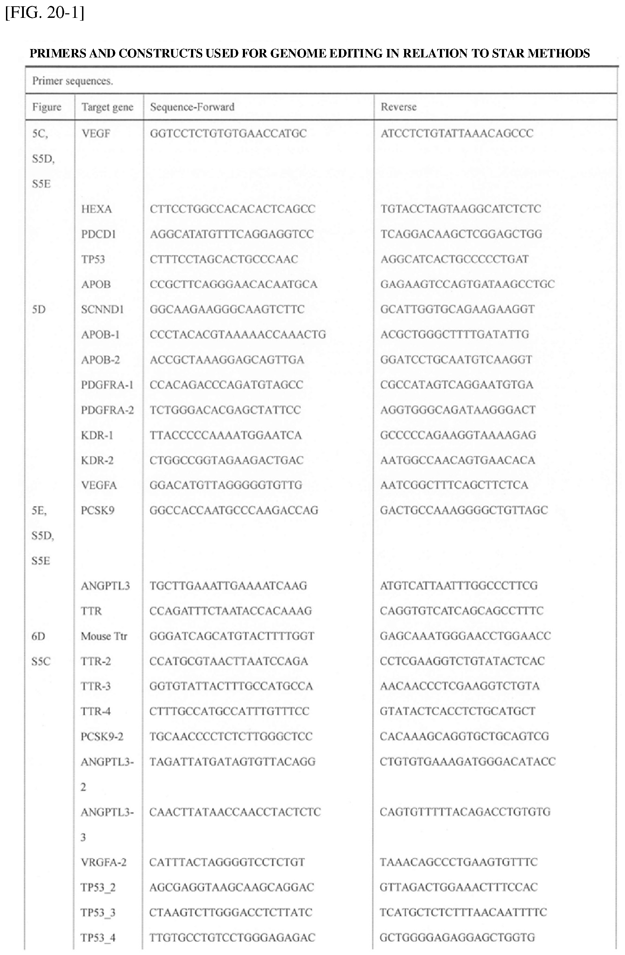

[FIG. 20-1] An explanatory table of primers and constructs used for genome editing in relation to STAR Methods.

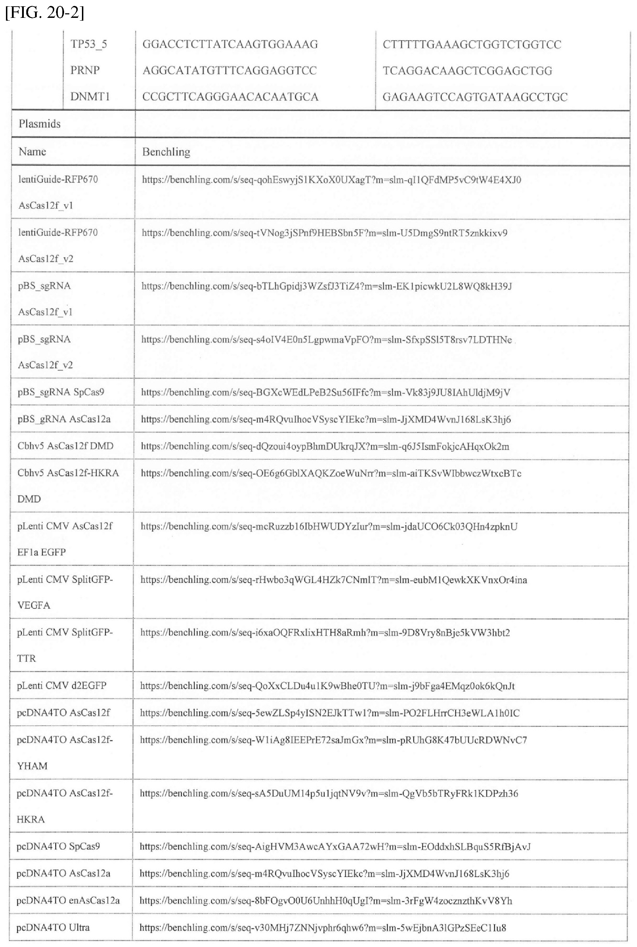

[FIG. 20-2] An explanatory table of the primers and the constructs used for the genome editing in relation to STAR Methods.

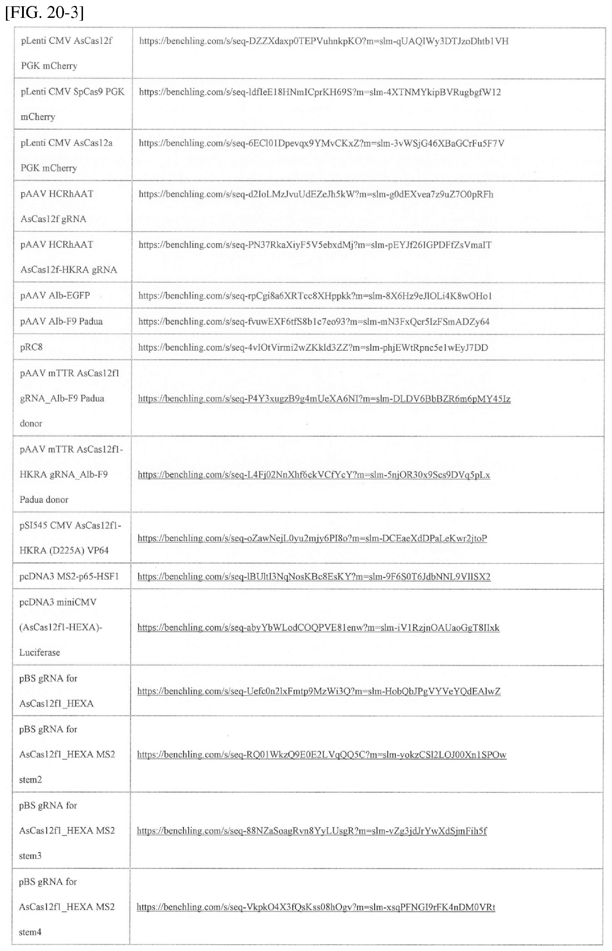

[FIG. 20-3] An explanatory table of the primers and the constructs used for the genome editing in relation to STAR Methods.

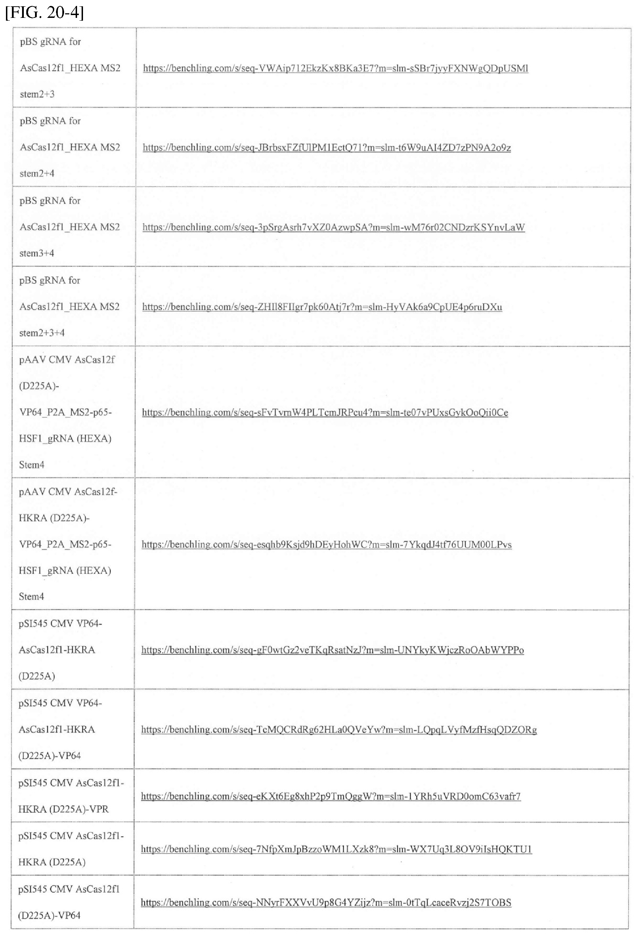

[FIG. 20-4] An explanatory table of the primers and the constructs used for the genome editing in relation to STAR Methods.

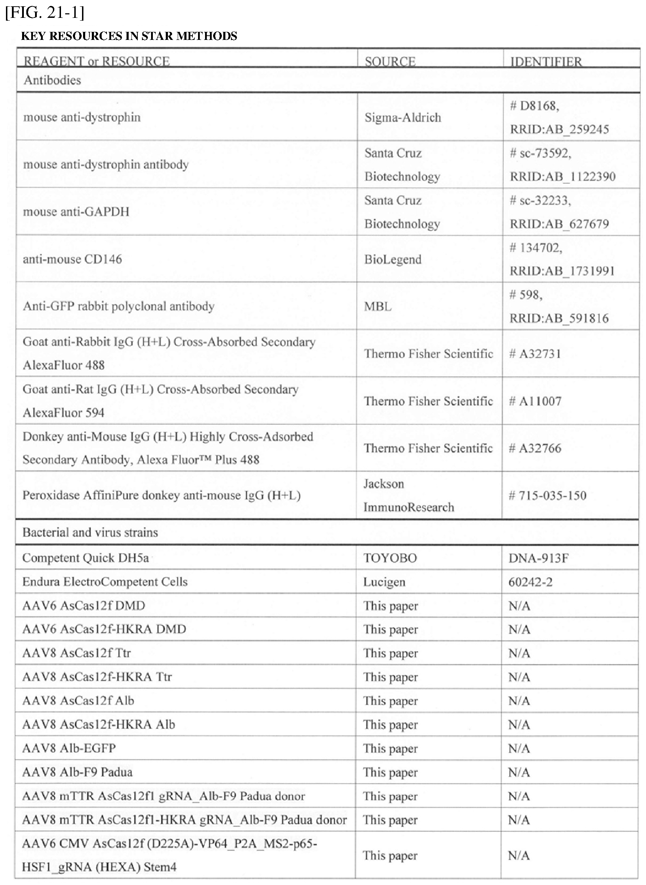

[FIG. 21-1] An explanatory table of key resources in STAR Methods.

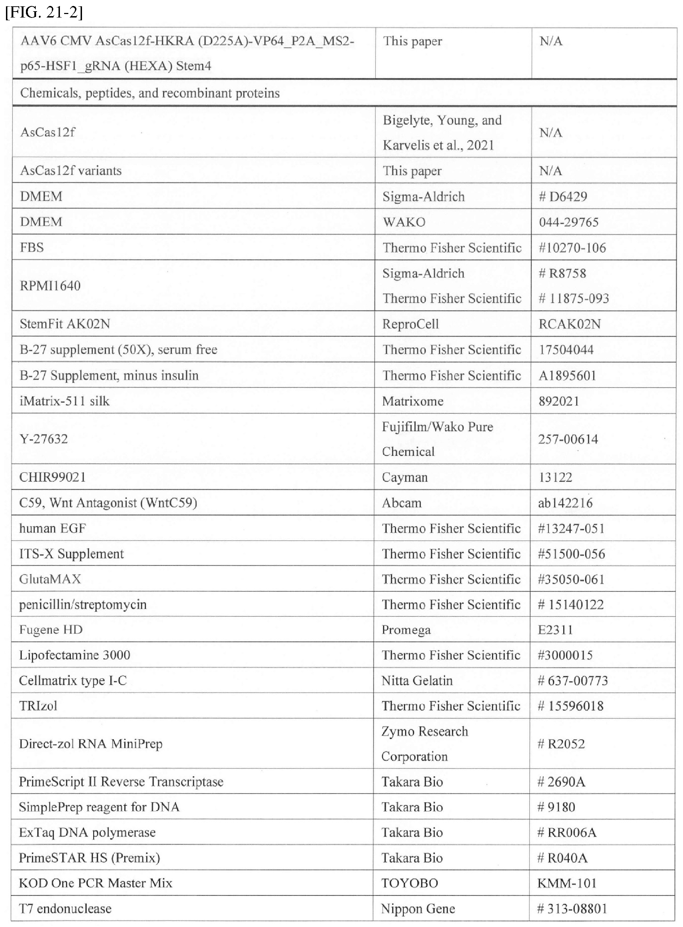

[FIG. 21-2] An explanatory table of the key resources in STAR Methods.

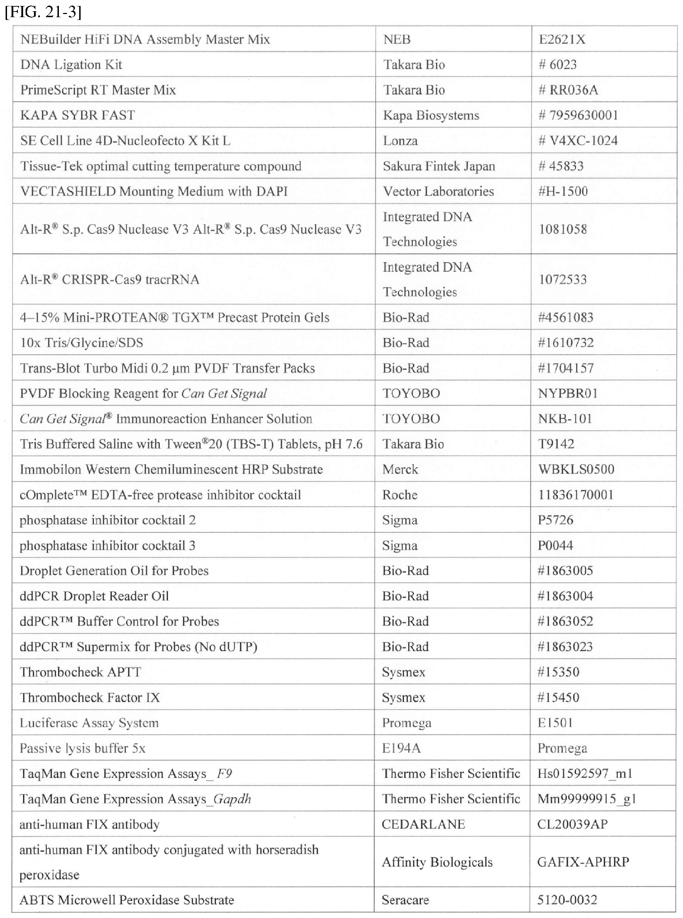

[FIG. 21-3] An explanatory table of the key resources in STAR Methods.

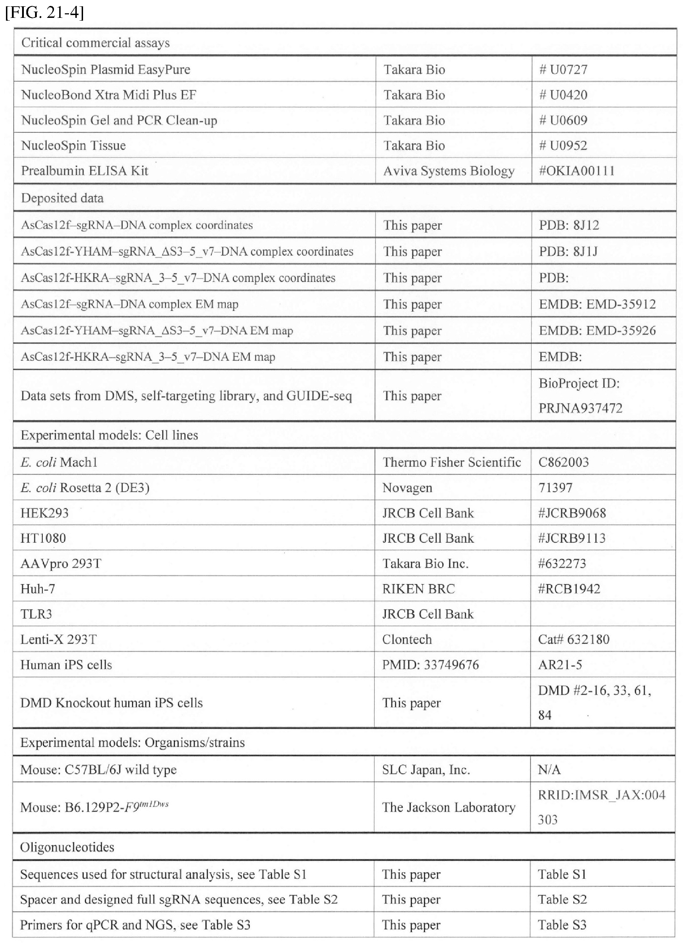

[FIG. 21-4] An explanatory table of the key resources in STAR Methods.

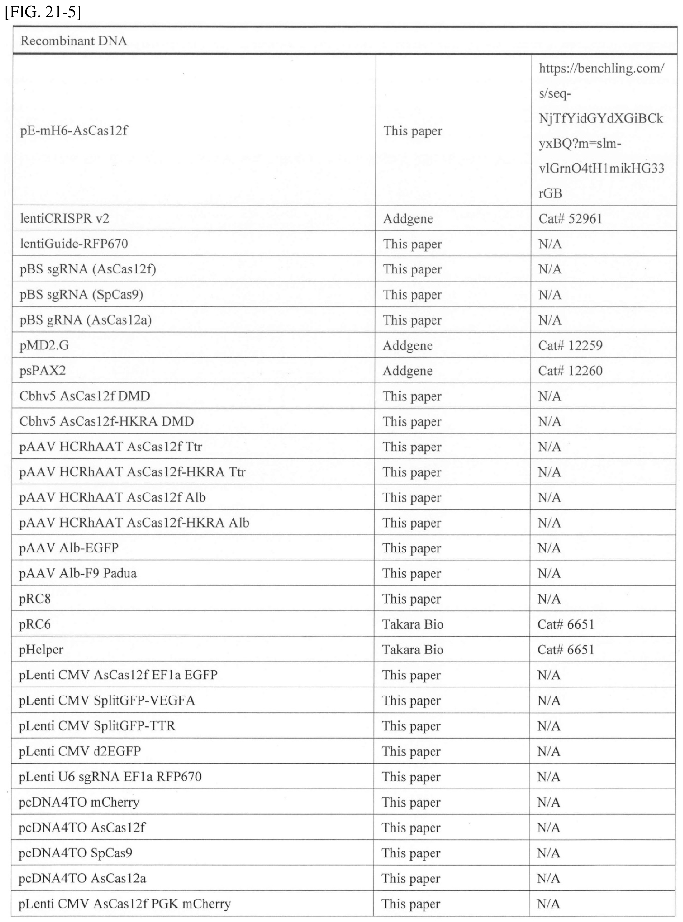

[FIG. 21-5] An explanatory table of the key resources in STAR Methods.

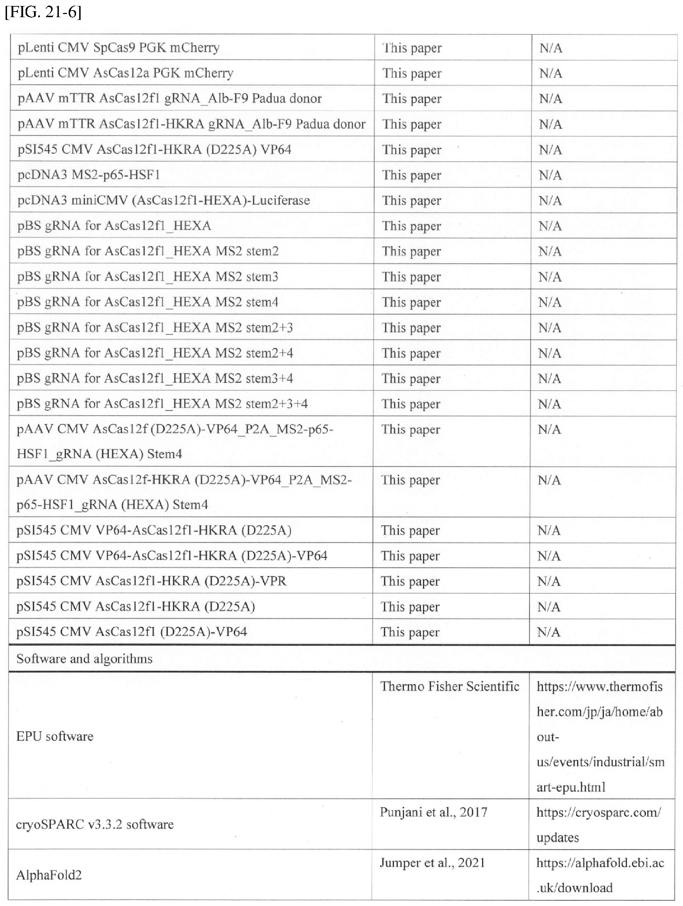

[FIG. 21-6] An explanatory table of the key resources in STAR Methods.

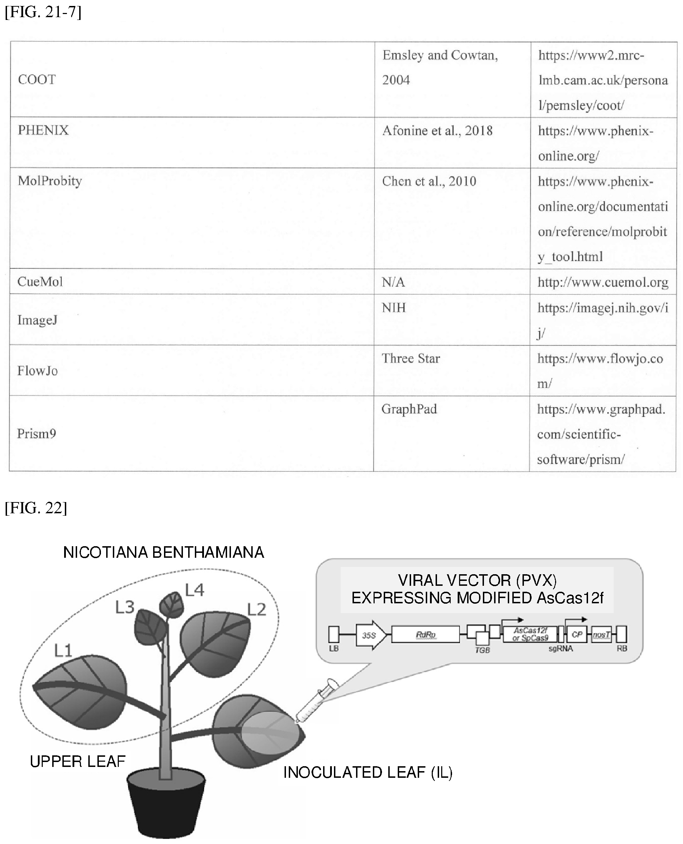

[FIG. 21-7] An explanatory table of the key resources in STAR Methods.
[FIG. 22] A schematic explanatory view of the genome editing of *Nicotiana benthamiana* with a viral vector expressing an AsCas12f variant.

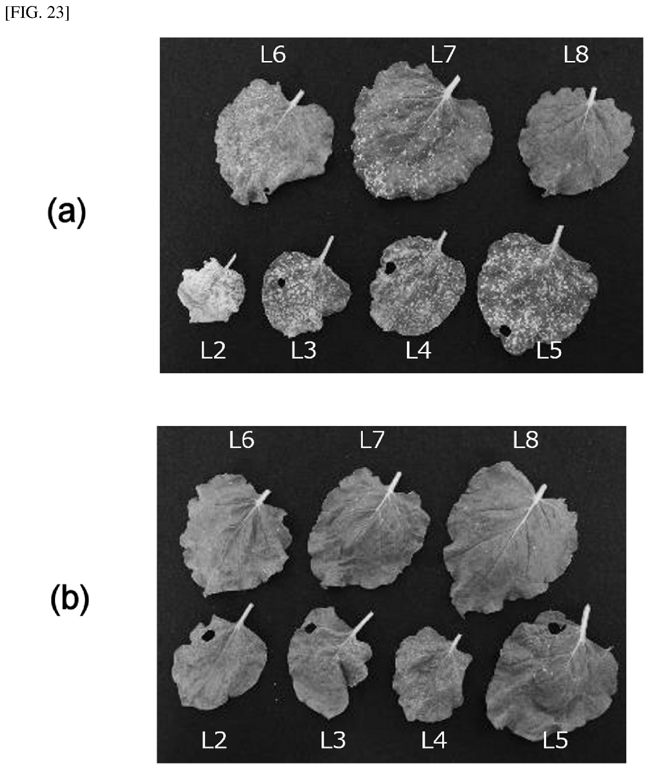

[FIGS. 23] FIG. 23(a) and FIG. 23(b) are explanatory photographs of the results of the visualization of genome-edited cells.

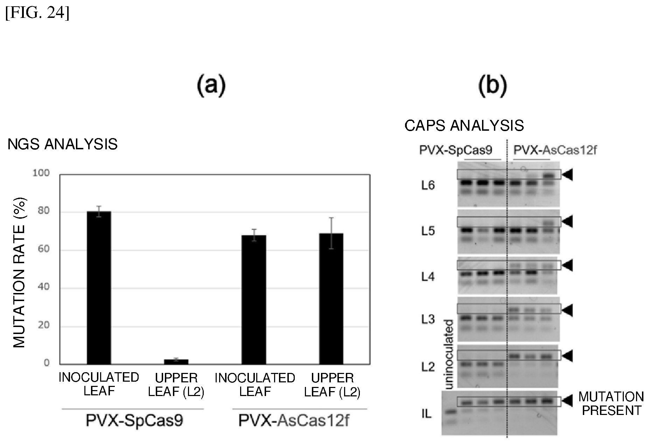

[FIGS. 24] FIG. 24(a) is an explanatory graph of NGS analysis results in the case where a PVX-AsCas12f variant and PVX-SpCas9 are each used, and FIG. 24(b) is an explanatory view of CAPS analysis results.

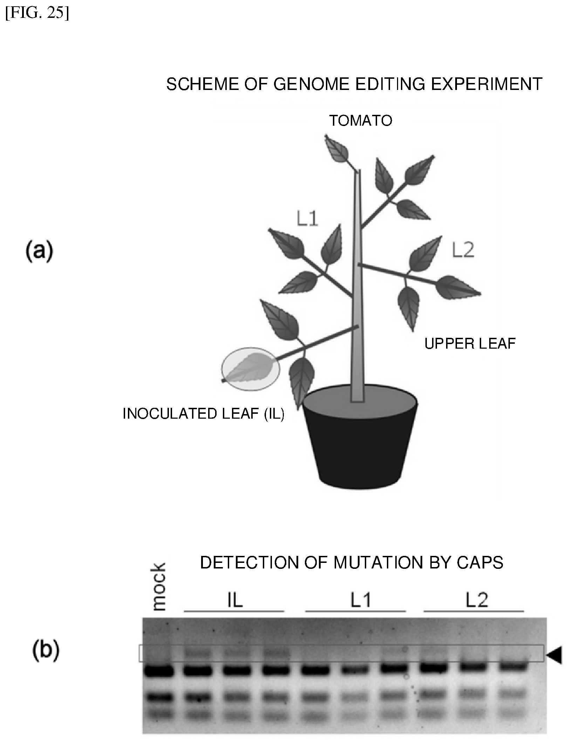

[FIGS. 25] FIG. 25(a) is a schematic explanatory view of the genome editing of tomato with a viral vector expressing an AsCas12f variant, and FIG. 25(b) is an explanatory view of CAPS analysis results with a PVX-AsCas12f variant.

### Description of Embodiments

In this description, when a substitution mutation in an amino acid sequence is represented, the mutation may be expressed as follows: the original amino acid is noted with a single letter; subsequently, a position number formed of 1 to 3 digits is attached thereto; and then the substituted amino acid is noted with a single letter. For example, when a mutation in which aspartic acid (D) is substituted with asparagine (N) occurs at amino acid position 1,022, the mutation is represented as "D1022N", which is synonymous with "substitution of Asp at amino acid position 1,022 with Asn." A -CO-NH- bond connecting amino acids is called a peptide bond. Amino acids bonded in this manner are referred to as "amino acid residues." In addition, the term "mutation" refers to a change that has occurred in a starting amino acid or nucleic acid sequence. The term is intended to encompass substitutions, insertions, and deletions. Further, in this embodiment, an engineered substance is a protein such as engineered AsCas12f. The engineered substance may be used as a composition together with any other substance, such as guide RNA or a vector.

Bacterial and archaeal CRISPR-Cas systems each provide adaptive immunity against a foreign nucleic acid, and are classified into two classes (Classes 1 and 2) and six types (Types I to VI). The Class 2 system includes Types II, V, and VI, and includes a single multi-domain effector Cas protein, such as Cas9 (Type II) or Cas12 (Type V).

The applications of CRISPR-Cas are not limited to genome editing. A Cas in which cleavage activity has been eliminated functions as a RNA-dependent DNA-binding protein, and hence has been used in technologies, such as the adjustment of transcriptional activity and single-base editing, by being fused with various effectors.

In genome editing enzymes, viral vectors such as an AAV are used for their introduction into target tissues. However, the size of a gene that can be loaded is limited, and Cas9 and Cas12a are difficult to load because of their sizes.

Cas9 binds to a dual RNA guide (CRISPR RNA [crRNA] and trans-activating crRNA [tracrRNA]) or single guide RNA (sgRNA), and cleaves a double-stranded DNA (dsDNA) target at a sequence, which is complementary to the 20-nt guide segment of the RNA guide and is adjacent to an NGG (N represents any nucleotide) proto-spacer adjacent motif (PAM).

Of various Type V Cas12s, Type V-A Cas12a (also known as Cpf1) binds to the crRNA and cleaves the dsDNA target at a TTTV (V represents A, G, or C) PAM. Cas9 includes two nuclease domains HNH and RuvC, which cleave the target strand (TS) and non-target strand (NTS) of the dsDNA target, respectively.

In contrast, Cas12a cleaves both the TS and the NTS with a single RuvC nuclease domain. Cas9 and Cas12a have been widely used as versatile genome engineering tools because the proteins each exhibit strong nuclease activity in a eukaryotic cell.

In addition, Cas9 (Type II) derived from *Streptococcus pyogenes* (SpCas9) and Cas12a from *Acidaminococcus* sp. (AsCas12a) out of various Type V Cas12s have been widely utilized as genome editing tools in human cells.

However, those proteins have relatively large sizes, and the sizes serve as limitations in delivery by adeno-associated virus vectors that are limited in terms of cargo size. Meanwhile, Type V-F Cas12f derived from *Acidibacillus sulfuroxidans* (AsCas12f) is extremely compact (422 amino acids) and functions in human cells. Table 1 shows the results of size comparison between AsCas12f and Cas reported so far. AsCas12f has an amino acid sequence as set forth in SEQ ID NO: 1 and includes 422 residues, the size being the smallest among the currently reported Cas. Accordingly, it can be seen that the size is one half or less of that of Cas9, Cas12a, Cas12b, Cas12e, or the like.

**Table 1**

| subtype | species | A.A. | |
|---|---|---|---|
| Cas9 | *S. pyogenes* | 1,368 _{(4.1 kb)} | |
| | *S. aureus* | 1,053 | |
| | *C*. *jejuni* | | 984 |
| Cas12a | *F. novicida* | 1,301 | |
| Cas12b | *A. acidoterrestris* | 1,129 | |
| Cas12e | *D. Bacterium* | | 986 |
| **Cas12f** | *A. sulfuroxidans* | | **422 _{(1.6 kb)}** |

However, the nuclease activity of wild-type AsCas12f is relatively low. In view of the foregoing, in this embodiment, two kinds of AsCas12f activity-enhanced (enAsCas12f) variants were developed by structural analysis with a cryo-electron microscope and variant screening.

In particular, the enAsCas12f variants each exhibited genome editing activity equivalent to or higher than that of SpCas9 or AsCas12a in human cells. As a result of structural analysis, it was revealed that each of the variants stabilized dimer formation and strengthened an interaction with a nucleic acid to improve DNA cleavage activity. Further, an all-in-one AAV vector in which partner genes were packaged together exhibited efficient knock-in/knock-out activity and transcriptional activation in mice. All things considered, enAsCas12f may provide a minimal genome editing platform for in vivo gene therapy.

The Cas (clustered regularly interspaced short palindromic repeats and CRISPR-associated proteins) systems, which provide adaptive immunity against the mobile genetic elements of bacteria and archaea, are classified into two classes (Class 1 and Class 2) and six types (Types I-VI). SpCas9 is accompanied by a dual RNA guide (CRISPR RNA [crRNA] and trans-activating crRNA [tracrRNA], or artificially linked single guide RNA [sgRNA]), and cleaves a double-stranded DNA (dsDNA) target flanked by NGG (N represents any nucleotide) proto-spacer adjacent motifs (PAMs) by using its HNH and RuvC nuclease domains. In contrast, AsCas12a binds to the crRNA, and cleaves the dsDNA target having a TTTV (V represents A, G, or C) PAM by using a single RuvC nuclease domain.

SpCas9 and AsCas12a each exhibit robust nuclease activity in a eukaryotic cell, and these proteins have been widely used as versatile genome engineering tools. However, SpCas9 (1,368 amino acids) and AsCas12a (1,307 amino acids) each have a large gene size, and hence it is difficult to efficiently package such protein into a single adeno-associated virus (AAV) vector. The difficulty hinders the clinical application of the proteins to in vivo gene therapy.

It has been shown that Type V-F Cas12 effectors are extremely compact (400 to 700 amino acids) RNA-guided DNA endonucleases. Cas12f associates with a dual crRNA:tracrRNA guide and cleaves target DNA having a T-rich PAM. Previous structural studies of Cas12f derived from an uncultured archaeon (UnCas12f), which has 529 amino acids, have revealed that UnCas12f functions as a dimer in order to compensate for its small size. However, UnCas12f has limitations in its application as a genome editing tool because UnCas12f cleaves DNA targets only under low-salt conditions in vitro and lacks activity in human cells. Meanwhile, as described below, the smallest Cas12f derived from *Acidibacillus sulfuroxidans* (AsCas12f) includes only 422 amino acids, and can cleave a DNA target having a TTR (R represents A or G) PAM under physiological conditions in vitro. Further, AsCas12f exhibits genome editing activity that is small but detectable in human cells. Accordingly, AsCas12f is useful as a small genome editing tool that can be packaged into a single AAV vector.

In this embodiment, an AsCas12f system for genome editing was improved. The combination of structural analysis and deep mutational scanning (DMS) elucidated the molecular basis of the system, and identified a detailed landscape of amino acid substitutions that significantly enhanced the nuclease activity of AsCas12f. Synergy between those mutations and guide RNA engineering significantly improved the genome editing efficiency of AsCas12f in human cells to a level comparable to or higher than those of SpCas9 and an engineered Cas12a protein. The size of AsCas12f is compact, and the compactness is extremely useful for AAV-deliverable gRNAs and the partner genes of, for example, base editors and epigenome modifiers. Accordingly, the AsCas12f system can be a promising genome editing platform.

### <Cryo-electron Microscopy Structure of AsCas12f-sgRNA-target DNA Ternary Complex>

FIG. 1-1 is a view for illustrating the domain structure of AsCas12f (A) and the cryo-electron microscopy map of an AsCas12f-sgRNA-target DNA ternary complex (B). FIG. 1-2 is a view for illustrating the structural model of the AsCas12f-sgRNA-target DNA ternary complex (C). FIG. 1-3 is a schematic view of sgRNA and target DNA (D), and in the figure, a disordered region is surrounded by a dashed frame, TS represents a target strand, NTS represents a non-target strand, and PK represents a pseudoknot. In order to distinguish the NTS and the TS, the NTS is indicated with an asterisk.

FIG. 2-1 and FIG. 2-2 are graphs and photographs showing the cryo-electron microscopy analysis of AsCas12f-sgRNA-target DNA (A to D), FIG. 2-3 and FIG. 2-4 are graphs and photographs showing the cryo-electron microscopy analysis of AsCas12f-YHAM-sgRNA_ΔS3-5_v7-target DNA (E to H), and FIG. 2-5 and FIG. 2-6 are graphs and photographs showing the cryo-electron microscopy analysis of an AsCas12f-HKRA-sgRNA_ΔS3-5_v7-target DNA complex (I to L).

Specifically, in FIG. 2-1 to FIG. 2-6, Part A shows a profile of the size-exclusion chromatography of the AsCas12f-sgRNA-target DNA, Part E shows a profile of the size-exclusion chromatography of the AsCas12f-YHAM-sgRNA_ΔS3-5_v7-target DNA, and Part I shows a profile of the size-exclusion chromatography of the AsCas12f-HKRA-sgRNA_ΔS3-5_v7-target DNA complex. The fractions of peaks were used for the following cryo-electron microscopy analysis.

In addition, in FIG. 2-1 to FIG. 2-6, Parts B, F, and J show typical cryo-electron microscopy images recorded on Titan Krios at 300 kV with a K3 camera. Parts C, G, and K are each an explanatory view of a single-particle cryo-electron microscopy image processing workflow. Parts D, H, and L are graphs showing Fourier shell correlation (FSC) curves for 3D reconstruction with a gold-standard cut-off indicated by black dotted lines (FSC=0.143).

As shown in Parts A to C of FIG. 1-1 and FIG. 1-2, Parts A to D of FIG. 2-1 and FIG. 2-2, and FIG. 9 and FIG. 10 showing the results of data collection, processing, model refinement, and validation, in order to understand the molecular mechanism of AsCas12f, the complex structure of AsCas12f with 222-nucleotide (nt) sgRNA and 38-nt dsDNA in which a DNA backbone around a cleavage site was modified with phosphorothioate at a TTA PAM was analyzed with a cryo-electron microscope (cryo-EM), and was reconstructed at an overall resolution of 3.1 angstroms (Å). It was revealed from the structure that an asymmetric homodimer was formed by the assembly of two AsCas12f molecules (AsCas12f.1 and AsCas12f.2) and one sgRNA molecule.

The Cas12f dimer adopts a bi-lobed structure having a recognition (REC) lobe and a nuclease (NUC) lobe, and a guide RNA-target DNA heteroduplex is bound to a central channel between the two lobes. The REC lobe includes the wedge (WED) domains and REC domains of AsCas12f.1 and AsCas12f.2 (WED.1/WED.2/REC.1/REC.2), and the NUC lobe includes the RuvC domains and target nucleic acid binding (TNB) domains of AsCas12f.1 and AsCas12f.2 (RuvC.1/RuvC.2/TNB.1/TNB.2). In addition, the AsCas12f-sgRNA-DNA complex structure was found to function as a dimer as in UnCas12f.

As illustrated in Part D of FIG. 1-3, the sgRNA includes a 20-nt guide segment (G1 to C20) and a 202-nt sgRNA scaffold (A(-202) to C(-1)). The sgRNA scaffold has an unexpected architecture having two pseudoknots (PK1 and PK2) and five stems (Stem 1 to Stem 5), which are not predicted from its primary sequence. Stem 1 has a 6-base pair (bp) duplex (U(-192):A(-175) to G(-187):C(-180)), and PK1 includes a 4-bp duplex (U(-185):A(-2) to A(-182):U(-5)).

Stem 2 includes a 16-bp duplex (from G(-171):U(-125)) including two non-canonical base pairs (G(-171):U(-125) to G(-161):U(-135)), and Stem 3 includes three base pairs (G(-118):C(-109) to C(-116):G(-111)). PK2 has two base pairs (G(-122):C(-88) and G(-121):C(-87)), and connects Stems 2 and 3. Stem 4 includes a 16-bp duplex in which two duplexes (U(-102):A(-80) to C(-96):G(-86)) are coaxially stacked. As illustrated in Part D of FIG. 1-3, in particular, C(-103) positioned between Stems 3 and 4 is inverted (flipped out), and is inserted into Stem 1 and PK1 to form a base pair with G(-181). Thus, a continuous helix including Stem 1 and PK1 is formed. As illustrated in FIG. 1-3, a 5' region (A(-202):A(-194)) and Stem 5 (U(-70):U(-15)) are disordered in the structure of the sgRNA scaffold, and the disorder suggests their flexibility.

In FIG. 3-1 to FIG. 3-3, Part A of FIG. 3-1 is an illustration of structural comparison between AsCas12f and UnCas12f derived from an uncultured archaeon (PDB ID: 7C7L). Part B of FIG. 3-2 is an illustration of a dimer interface between AsCas12f.1 and AsCas12f.2, and recognition sites for a sgRNA scaffold. Part C of FIG. 3-2 is an illustration of the superposition of AsCas12f.1 and AsCas12f.2 based on a REC lobe. Part D of FIG. 3-3 is an illustration of a dimer interface between REC.1 and REC.2, and Part E of FIG. 3-3 is an illustration of a dimer interface between RuvC.1 and RuvC.2. Part F of FIG. 3-3 is an illustration of the recognition of Stem 2 by AsCas12f.2, and Part G of FIG. 3-3 is an illustration of the recognition of Stem 2 by AsCas12f.1.

AsCas12f (422 residues) is smaller than UnCas12f (529 residues) by 100 residues or more. Meanwhile, its cognate sgRNA is longer than that of UnCas12f by about 40 nucleotides. As illustrated in Part A of FIG. 3-1, the structural comparison between AsCas12f and UnCas12f has revealed that although their domain configurations are similar, AsCas12f lacks a zinc finger (ZF) domain inserted between a WED domain and a REC domain seen in UnCas12f. Instead, in the structure of AsCas12f, the ZF domain is replaced with PK2 and Stem 3 that are not present in UnCas12f. Those findings describe the miniaturization of AsCas12f, and the miniaturization is compensated by longer sgRNA.

### <Small AsCas12f Scaffold associated with Large sgRNA>

As illustrated in Parts B to E of FIG. 3-2 and FIG. 3-3, AsCas12f.1 and AsCas12f.2 interact with each other primarily through hydrophobic interactions at REC.1/REC.2 and RuvC.1/RuvC.2 interfaces. Thus, as observed in UnCas12f, the dimerization of AsCas12f is promoted. As illustrated in Parts D and E of FIG. 3-3, REC.1 and REC.2 form a symmetrical interface in which the domains face opposite directions to each other while W43, F48, and I116 play central roles. Meanwhile, RuvC.1 and RuvC.2 form an asymmetrical interface in which their respective protomers adopt different conformations. In particular, as illustrated in Part E of FIG. 3-3, the α1 helix and α1-α2 loop of RuvC.1 interact with the α1 helix and α2 helix of RuvC.2. As illustrated in Parts F and G of FIG. 3-3, in addition to protein-protein interactions, the lower and upper regions of Stem 2 interact extensively with AsCas12f.1 and AsCas12f.2, respectively. The foregoing suggested that protein-nucleic acid interactions also contributed to the dimerization of AsCas12f.

Part A of FIG. 4-1 is an illustration of recognition sites for guide RNA and target DNA, and Part B of FIG. 4-1 is an illustration of the electrostatic surface potential of AsCas12f. The heteroduplex of sgRNA and the target DNA, and the Stem 1, Stem 2, and PK1 regions of a sgRNA scaffold are accommodated within a positively charged groove of an AsCas12f dimer. Parts C to E of FIG. 4-2 are illustrations of the recognition of Stem 1 and PK1, Stem 3, and a guide RNA-target DNA heteroduplex, respectively. Part F of FIG. 4-3 is an illustration of the structural comparison of the TS, NTS, and RuvC active sites of AsCas12f with UnCas12f derived from an uncultured archaeon (PDB ID: 7C7L). The positions of RuvC.1 active sites relative to the target DNA are similar in both structures. The similarity suggests that AsCas12f also cleaves the target DNA with its RuvC.1 domain.

As illustrated in Parts A and B of FIG. 4-1, the assembly of the sgRNA scaffold and AsCas12f is facilitated by both of a base-specific interaction and a non-specific interaction. As illustrated in Part C of FIG. 4-2, a continuous helix including Stem 1 and PK1 is mainly recognized and accommodated within a groove formed by WED.1 and RuvC.1 domains. As illustrated in Parts F and G of FIG. 4-3, Stem 2 may pass through a gap between AsCas12f.1 and AsCas12f.2, and interact extensively with both protomers to enhance the dimerization of AsCas12f as described above. As illustrated in Part D of FIG. 4-2, PK2 is recognized by WED.1 and REC.1 domains through sugar-phosphate backbone interactions, and is further stabilized by the coordination of a metal ion. In contrast, Stems 3 and 4 are exposed to a solvent, and their interactions with AsCas12f are minimal.

As illustrated in Part B of FIG. 4-1 and Part E of FIG. 4-2, the heteroduplex of the guide RNA and the target DNA is accommodated within a positively charged central channel, and is recognized by AsCas12f through a non-specific interaction between bases. As illustrated in Part E of FIG. 4-2, the PAM-proximal region (G1:dC18 to G12:dC7) of the heteroduplex is mainly recognized by Cas12f.1, but the PAM-distal region (C13:dG6 to G18:dC1) of the heteroduplex is recognized by AsCas12f.2. As illustrated in Part E of FIG. 4-2, in particular, P240.2 of RuvC.2 is stacked with the G18:dC1 base pair of the heteroduplex. The stacking means that 18 nucleotides of a spacer sequence function as a guide segment. As illustrated in Part F of FIG. 4-3, the structural comparison with UnCas12f has revealed that the position of RuvC.1 of AsCas12f is similar to that of RuvC.1 of UnCas12f that cleaves both of a target strand and a non-target strand. It is suggested from those structural observations that AsCas12f.1 is responsible for the cleavage of the target DNA, and in contrast, AsCas12f.2 plays an important role in the recognition of the PAM-distal region of the heteroduplex.

### <Engineering of AsCas12f for improving Genome Editing Efficiency in Mammalian Cells>

AsCas12f has been clarified to exhibit only limited genome editing activity in human cells. In order to expand the utility of the ultra-compact protein, activity-enhanced AsCas12f variants were produced. Deep mutational scanning (DMS) was performed to examine how every amino acid substitution affected the genome editing efficiency of a HEK293T cell. Specifically, a mutation in which all the positions of the 422 residues of AsCas12f were changed to 20 kinds of amino acids was evaluated for how its activity changed in genome editing targeting a green fluorescent protein (GFP) by using the DMS (genome editing efficiency was judged by the loss of GFP fluorescence).

In FIG. 5-1, Part A is an explanatory view of a DMS library design for AsCas12f, and Part B is a view for illustrating the overview of a DMS approach for the genomic evaluation of gene editing efficiency in the context of GFP gene deletion. In FIG. 5-2, Part C is an explanatory graph of how every single mutation affects genome editing activity. Part D is an explanatory view of a Split-GFP-reconstitution assay for evaluating genome editing activity against a VEGF gene in HEK293T cells. Plasmid-encoded mCherry is co-infected to identify plasmid-infected cells. In addition, in FIG. 5-2, Part E is a view for illustrating the genome editing activity of AsCas12f mutations carrying editing efficiency-enhancing mutations determined by flow cytometry (n=4), and Part F is a graph showing the temporal course (n=3, mean±SD) of GFP deletion induced by AsCas12f-HKRA and AsCas12f in HEK293T cells each expressing d2EGFP. sgRNA_ΔS5 and sgRNA_ΔS3-5_v7 were transduced by using single-copy lentivirus infection. In the graph of Part F of FIG. 5-2, (a) shows results in HKRA+ΔS3-5_v7, (b) shows results in HKRA+ΔS5, (c) shows results in WT+ΔS3-5_v7, and (d) shows results in WT+ΔS5.

As illustrated in Part A of FIG. 5-1, a plasmid expressing both an EGFP and AsCas12f was designed, and an AsCas12f library covering all the 20 amino acid substitutions of the entire sequence (M1-K422) was created. As illustrated in Part B of FIG. 5-1, sgRNA targeting a GFP was introduced into HEK293T cells, and thereafter, an AsCas12f library packaged in a lentivirus was expressed at a multiplicity of infection (MOI) of less than 0.2 to ensure that the number of variant AsCas12f.1 molecules expressed per cell did not exceed 1. A Stem 5-deleted variant (sgRNA_ΔS5) with enhanced genome editing in human cells was used for screening because the genome editing efficiency of full-length sgRNA was not optimal.

Lentivirus-infected cells were selected by sorting GFP-positive cells on day 2 after the infection, and were further cultured for 5 days. As illustrated in Part B of FIG. 5-1, on day 7 after the infection, mRNAs were separately extracted from the GFP-positive cells and GFP-negative cells, and deep sequencing analysis was performed to identify mutations. Part C of FIG. 5-2 represents genome editing efficiency with a bar graph and a density distribution. The bar graph becomes longer and its color becomes darker in the density distribution as the genome editing efficiency increases as compared to that of a wild type. In Part C of FIG. 5-2, the density distribution is schematic, and the editing efficiency is represented mainly by the bar graph. As shown in the figure, the editing efficiency of each mutation was defined as the ratio of a GFP-negative read count to a total read count, and was normalized with the value of wild-type AsCas12f (WT: wild type).

Part A in FIG. 6-1 is a graph showing a correlation between the effects of single variants on GFP deletion determined from independent mutation library replicates. Part B-1 in FIG. 6-2 and Part B-2 in FIG. 6-3 are the lists of single amino acid substitutions in each of which editing efficiency increases by 20% or more as compared to that of the WT. Part C in FIG. 6-4 is a graph showing the genome editing activity of each of AsCas12f variants when the variants are double, Part D therein is a graph showing the genome editing activity when the variants are triple, and Part E therein is a graph showing the genome editing activity when the variants are quadruple.

As shown in Part A of FIG. 6-1, a DMS experiment was performed in duplicate, and similar results were obtained [coefficient of determination (R2)=about 0.6]. As a result, as shown in Parts B-1 and B-2 of FIG. 6-2 and FIG. 6-3, and Part C in FIG. 6-4, 200 or more amino acid substitutions in each of which the editing efficiency increased by 20% or more as compared to that of the WT were identified. In each of Parts B-1 and B-2, 208 mutations in each of which the genome editing efficiency is 1.2 times or more as high as that of the wild type are indicated on its axis of abscissa, and its axis of ordinate indicates the genome editing efficiency of each mutation when the genome editing efficiency of the wild type is 1. In Parts B-1 to E of FIG. 6-2 to FIG. 6-4, mutations in finally produced variants (F48Y, I123H, S188H, D195K, D208R, V232A, and E316M) are highlighted with diagonal lines.

Parts C, D, and E of FIG. 6-4, and Parts E and F of FIG. 5-2 show the results of the combination of mutations and the measurement of genome editing efficiency. In those measurements, in contrast to Parts B-1 and B-2 of FIG. 6-2 and FIG. 6-3, GFP fluorescence was restored by causing genome editing against a gene whose GFP fluorescence had been lost. Attention was paid to S188H and D195K out of those mutations. Those mutations are positioned near nucleic acids in the structure of the gene, and may each form an additional interaction for enhancing target DNA binding by AsCas12f. Accordingly, two highly active variants were finally developed by combining a plurality of mutations for each of S188H and D195K (Part E of FIG. 5-2).

Next, as illustrated in Part D of FIG. 5-2, a split-GFP reporter system linked with a frame-shift linker including a VEGFA sequence was developed, and the genome editing activity of each of the WT and several variants was evaluated by measuring the ratio of frame-shift GFP-positive cells. As shown in Part E of FIG. 5-2, an S188H variant and a D195K variant each exhibited genome editing activity higher than that of the WT, the results being consistent with their DMS analysis. In order to produce a variant having genome editing activity higher than that of a single S188H or D195K variant, 20 or more variants were produced by combining an S188H or D195K substitution and any other effective substitution identified in the DMS analysis.

As shown in Part E of FIG. 5-2 and Part C of FIG. 6-4, some double variants each exhibited a reduction in genome editing activity, but S188H/V232A and D195K/V232A each exhibited improved activity. In order to further improve the activity, seven triple variants were produced by additionally introducing a mutation that enhanced the activity when combined with S188H or D195K. As shown in Part E of FIG. 5-2 and Part D of FIG. 6-4, triple variants S188H/V232A/E316M and D195K/D208R/V232A were each further improved in genome editing activity. Finally, 29 quadruple variants were constructed by combining the triple variant S188H/V232A/E316M or D195K/D208R/V232A with the other effective substituents identified in the DMS analysis, and were each evaluated for its genome editing activity. As shown in Part E of FIG. 5-2 and Part D of FIG. 6-4, most of those variants each exhibited activity equivalent to or lower than that of the corresponding triple variant, but quadruple variants F48Y/S188H/V232A/E316M and I123H/D195K/D208R/V232A were each further improved in genome editing activity. In this description, the F48Y/S188H/V232A/E316M and I123H/D195K/D208R/V232A variants were named AsCas12f-YHAM and AsCas12f-HKRA, respectively.

In addition, as shown in the graph of Part E of FIG. 5-2 , the ratios of the frame-shift GFP-positive cells in S188H, which is an example of a variant having no mutation at amino acid position 195, having a mutation at amino acid position 188, and having no mutations at the other amino acid positions, and in D195K, which is an example of a variant having no mutation at amino acid position 188, having a mutation at amino acid position 195, and having no mutations at the other amino acid positions, are each about 18%. Accordingly, a variant having a mutation at amino acid position 188 is shown to have a significantly higher ratio of the frame-shift GFP-positive cells as compared to a value in the WT that is a comparative example. In particular, S188H, which has a mutation to histidine (H) at amino acid position 188, is shown to have a significantly higher ratio of the frame-shift GFP-positive cells as compared to that of the WT. In addition, a variant having a mutation at amino acid position 195 is shown to have a higher ratio of the frame-shift GFP-positive cells as compared to that of the WT. In particular, D195K, which has a mutation to lysine (K) at amino acid position 195, is shown to have a significantly higher ratio of the frame-shift GFP-positive cells as compared to that of the WT.

In contrast, in S188A/V232A, which is an example of a double variant further having a mutation at amino acid position 232 in addition to the mutation at amino acid position 188 under such a condition that there is no mutation at amino acid position 195, the value of the ratio is significantly improved because the number of the frame-shift GFP-positive cells is slightly less than twice as large as that in S188H. Further, in S188H/V232A/E316M, which is an example of a triple variant having a mutation at amino acid position 316 in addition to the mutations at amino acid position 188 and amino acid position 232, the value is further improved because the number of the frame-shift GFP-positive cells is about 2.5 times as large as that in S188H.

Further, in F48Y/S188H/V232A/E316M, which is an example of a quadruple variant having a mutation at amino acid position 48 in addition to the mutations at amino acid position 188, amino acid position 232, and amino acid position 316, the value of the ratio is further improved because the number of the frame-shift GFP-positive cells is slightly less than three times as large as that in S188H. In addition, it is strongly estimated from those results that a multi-variant, such as a quintuple variant or a sextuple variant including the mutations of F48Y/S188H/V232A/E316M, also has a higher ratio of the frame-shift GFP-positive cells as compared to that of the WT. The foregoing shows that variants each having a mutation at amino acid position 188, in particular, a variant including only the mutation of S188H, and any multi-variant, such as a double variant, a triple variant, or a quadruple variant including at least the mutation of S188H, are useful. Accordingly, the following important finding has been obtained: the ratio of the frame-shift GFP-positive cells tends to be significantly improved in the case of a variant having a mutation at amino acid position 188.

In addition, in Part C of FIG. 6-4, in a double variant of the combination of S188H and D208R, although a high value of about three times as high as that of the WT is obtained, the value is lower than those of the other double variants. Further, as shown in Part E of FIG. 6-4, in a quadruple variant of the combination of S188H, V232A, E316M, and D208R, although a high value of about six times as high as that of the WT is obtained, the value is lowest as compared to those of the other quadruple variants.

Accordingly, it is strongly estimated that out of double variants, triple variants, quadruple variants, quintuple variants, sextuple variants, ···, and m-fold variants ("m" represents a natural number of 2 or more) each including the mutation of S188H, a variant that is free of the mutation of D208R has a high ratio of the frame-shift GFP-positive cells and hence provides even higher genome editing activity.

In addition, in D195K/V232A, which is an example of a double variant further having a mutation at amino acid position 232 in addition to the mutation at amino acid position 195 under such a condition that there is no mutation at amino acid position 188, the number of the frame-shift GFP-positive cells is slightly less than twice as large as that in D195K, and hence the value of their ratio is significantly improved. Further, in D195K/D208R/V232A, which is an example of a triple variant having a mutation at amino acid position 208 in addition to the mutations at amino acid position 195 and amino acid position 232, the number of the frame-shift GFP-positive cells is about 2.5 times as large as that in D195K, and hence the value of their ratio is further improved.

Further, in I123H/D195K/D208R/V232A, which is an example of a quadruple variant having a mutation at amino acid position 123 in addition to the mutations at amino acid position 195, amino acid position 232, and amino acid position 208, the number of the frame-shift GFP-positive cells is slightly less than three times as large as that in D195K, and hence the value of their ratio is further improved. In addition, it is strongly estimated from those results that a multi-variant, such as a quintuple variant or a sextuple variant including the mutations of I123H/D195K/D208R/V232A, also has a higher ratio of the frame-shift GFP-positive cells as compared to that of the WT. The foregoing shows that variants each having a mutation at amino acid position 195, in particular, a variant including only the mutation of D195K, and any multi-variant, such as a double variant, a triple variant, or a quadruple variant including at least the mutation of D195K, are useful. Accordingly, the following important finding has been obtained: the ratio of the frame-shift GFP-positive cells tends to be significantly improved in a variant having a mutation at amino acid position 195.

Thus, a variant including at least the mutation of S188H and a variant including at least the mutation of D195K are shown to be particularly useful. In addition, as shown in the graph of Part C of FIG. 6-4, the ratio of the frame-shift GFP-positive cells in a double variant including both the mutations of S188H and D195K (S188H/D195K) is about 25%. As shown in Part C of FIG. 6-4, a value higher than those in a variant including only the mutation of S188H and a variant including only the mutation of D195K is obtained.

Meanwhile, as shown in Part D of FIG. 6-4, in each of triple variants each including at least the mutations of S188H and V232A without a mutation at amino acid position 195 (e.g., S188H/V232A/E316M), the ratio of the frame-shift GFP-positive cells is a value of from about 40% to about 50%. The value is significantly higher than a value in a variant having only the mutation of S188H, and a value in a double variant including both the mutations of S188H and D195K. Accordingly, it is shown that those triple variants are particularly useful. Further, as shown in Part E of FIG. 6-4, in each of a triple variant including the mutations of S188H, V232A, and E316M (S188H/V232A/E316M), and a quadruple variant including at least the mutations of S188H, V232A, and E316M (e.g., S188H/V232A/E316M/F48Y), the ratio of the frame-shift GFP-positive cells is from about 20% to about 50%. Accordingly, it is shown that those variants are useful. In particular, quadruple variants S188H/V232A/E316M/I23K, S188H/V232A/E316M/F48Y, S188H/V232A/E316M/K80R, S188H/V232A/E316M/I123H, S188H/V232A/E316M/E246M, S188H/V232A/E316M/I262N, S188H/V232A/E316M/E354T, S188H/V232A/E316M/D364Q, and S188H/V232A/E316M/I388N each have a value higher than the value in the double variant including the mutations of S188H and D195K. Accordingly, it is shown that those variants are particularly useful.

In addition, as shown in Part D of FIG. 6-4, in each of triple variants each including at least the mutations of D195K and V232A without a mutation at amino acid position 188 (e.g., D195K/D208R/V232A), the ratio of the frame-shift GFP-positive cells is a value of from about 30% to about 50% or more. The value is significantly higher than a value in a variant having only the mutation of D195K, and a value in a double variant including the mutations of S188H and D195K. Accordingly, it is shown that those triple variants are particularly useful. Further, as shown in Part E of FIG. 6-4, in each of a triple variant including the mutations of D195K, V232A, and D208R (D195K/D208R/V232A), and a quadruple variant including at least the mutations of D195K, V232A, and D208R (e.g., D195K/D208R/V232A/I123H), the ratio of the frame-shift GFP-positive cells is from about 25% to about 50%. Accordingly, it is shown that those variants are useful.

In Part E of FIG. 6-4, the ratio of the frame-shift GFP-positive cells in D195K/D208R/V232A/S188R, which is a quadruple variant including a mutation at amino acid position 188, is about 25%, which is a value comparable to that in a double variant having both the mutations of S188H and D195K. However, in any other quadruple variant including the mutations of D195K/D208R/V232A, as shown on the right side of Part E of FIG. 6-4, a value as high as from about 35% to about 50% is obtained for the ratio of the frame-shift GFP-positive cells. It is shown from the foregoing that a quadruple variant having a mutation at amino acid position 195 without a mutation at amino acid position 188, in particular, a quadruple variant including at least the mutations of D195K/D208R/V232A without a mutation at amino acid position 188 is useful. In addition, not only quadruple variants but also quintuple variants and sextuple variants each including at least the mutations of D195K/D208R/V232A without a mutation at amino acid position 188 may be useful.

In addition, each of triple variants shown in Part D of FIG. 6-4 is a triple variant having a mutation at only one of amino acid position 188 or amino acid position 195. In addition, in each of those variants, the ratio of the frame-shift GFP-positive cells exceeds 30%. Accordingly, both of the following variants may be useful: (1) a triple variant including at least the mutations of S188H/V232A without a mutation at amino acid position 195; and (2) a triple variant including at least the mutations of D195K/V232A without a mutation at amino acid position 188. In addition, as long as the conditions (1) and (2) are satisfied, not only triple variants but also any multi-variants, such as quadruple variants and quintuple variants, may be useful.

It is shown from the foregoing that a protein having a mutation at amino acid position 188 in the amino acid sequence of AsCas12f significantly improves the ratio of the frame-shift GFP-positive cells. In addition, it is shown that a protein having a mutation at amino acid position 195 in the amino acid sequence of AsCas12f also significantly improves the ratio of the frame-shift GFP-positive cells.

More specifically, as shown on the left side of Part C of FIG. 6-4, in each of double variants from S188H/I2Y to S188H/L337I, the ratio of the frame-shift GFP-positive cells is a value significantly higher than the value (from about 3% to about 4%) in the WT that is a comparative example. In particular, although the ratio of the frame-shift GFP-positive cells in S188H/D208R is about 6% that is minimum, it is clearly shown that the value is significantly higher than that of the WT. It can be said from the foregoing that a double variant satisfying such a condition that there is a mutation to histidine at amino acid position 188 is useful.

In addition, in the graph regarding S188H in Part C of FIG. 6-4, in each of S188H/K80R, S188H/Y105T, S188H/I123H, S188H/D195K, S188H/V232A, S188H/E246M, S188H/E316M, and S188H/L337I, the ratio of the frame-shift GFP-positive cells is higher than that in the case of only the mutation of S188H. Accordingly, those variants are shown to be particularly useful double variants.

Next, as shown on the right side of Part C of FIG. 6-4, in each of double variants from D195K/I22Y to D195K/L337I, the ratio of the frame-shift GFP-positive cells is a value significantly higher than the value (from about 3% to about 4%) in the WT that is a comparative example. In particular, although the ratio of the frame-shift GFP-positive cells in D195K/Y105T is about 16% that is minimum, it is clearly shown that the value is significantly higher than that of the WT. It can be said from the foregoing that a double variant satisfying such a condition that there is a mutation to lysine at amino acid position 195 is useful.

In addition, in the graph regarding D195K in Part C of FIG. 6-4, in each of D195K/I2Y, D195K/N70Y, D195K/K80R, D195K/I123H, D195K/D208R, D195K/V232A, D195K/E246M, D195K/E316M, and D195K/L337I, the ratio of the frame-shift GFP-positive cells is higher than that in the case of only the mutation of S188H. Accordingly, those variants are shown to be particularly useful double variants.

Next, as shown in Part D of FIG. 6-4, the ratio of the frame-shift GFP-positive cells in each of triple variants S188H/V232A/I123H, S188H/V232A/E246M, S188H/V232A/E316M, and S188H/V232A/L337I is about 35% or more, which is a value significantly higher than the value in the WT that is a comparative example, and which is a value comparable to or higher than that in an S188H/V232A double variant. In particular, in the triple variants S188H/V232A/E246M and S188H/V232A/E316M, values higher than that in the S188H/V232A double variant are obtained. Accordingly, it is shown that those triple variants are useful. In addition, it can be said that a triple variant satisfying such a condition that the mutations of S188H/V232A are included is particularly useful because a high value is stably obtained for the ratio of the frame-shift GFP-positive cells as described above. In addition, as shown on the left side of Part E of FIG. 6-4, the ratio of the frame-shift GFP-positive cells is high also in each of quadruple variants each satisfying such a condition that the mutations of S188H/V232A are included. It is shown from the foregoing that not only triple variants but also n-fold variants ("n" represents a natural number of 3 or more) each including at least the mutations of D195K/V232A are useful.

In addition, the ratio of the frame-shift GFP-positive cells in each of triple variants D195K/V232A/N70Y, D195K/V232A/D206R, and D195K/V232A/E316M is about 30% or more, which is a value significantly higher than the value in the WT that is a comparative example, and which is a value comparable to or higher than that in a D195K/V232A double variant. In particular, in the triple variants S188H/V232A/E246M and S188H/V232A/E316M, values higher than that in the S188H/V232A double variant are obtained. Accordingly, it is shown that those triple variants are useful. In addition, it can be said that a triple variant satisfying such a condition that the mutations of D195K/V232A are included is particularly useful because a high value is stably obtained for the ratio of the frame-shift GFP-positive cells as described above. In addition, as shown on the right side of Part E of FIG. 6-4, the ratio of the frame-shift GFP-positive cells is high also in each of quadruple variants each satisfying such a condition that the mutations of D195K/V232A are included. It is shown from the foregoing that not only triple variants but also n-fold variants ("n" represents a natural number of 3 or more) each including at least the mutations of D195K/V232A are useful.

In each of the quadruple variants shown on the left side of Part E of FIG. 6-4, the variants each including the mutations of S188H/V232A/E316M, the ratio of the frame-shift GFP-positive cells is from about 20% to about 50%, which is a value significantly higher than the value in the WT that is a comparative example. In addition, in Part E of FIG. 6-4, although the ratio of the frame-shift GFP-positive cells in a quadruple variant S188H/V232A/E316M/D206 is about 20% that is minimum, it is clearly shown that the value is significantly higher than that of the WT. It is shown that a quadruple variant satisfying such a condition that the mutations of S188H/V232A/E316M are included is particularly useful because a high value is stably obtained for the ratio of the frame-shift GFP-positive cells as described above. Further, in each the quadruple variants S188H/V232A/E316M/F48Y, S188H/V232A/E316M/E246M, S188H/V232A/E316M/I262N, and S188H/V232A/E316M/I388N, the ratio of the frame-shift GFP-positive cells is comparable to or higher than that in the S188H/V232A/E316M triple variant. Accordingly, it is shown that those variants are particularly useful quadruple variants. In addition, it is strongly estimated from the foregoing that not only quadruple variants each including at least the mutations of S188H/V232A/E316M but also m-fold variants ("m" represents a natural number of 4 or more) each including at least the mutations of S188H/V232A/E316M are useful.

In each of the quadruple variants shown on the right side ofPart E of FIG. 6-4, the variants each including the mutations of D195K/D208R/V232A, the ratio of the frame-shift GFP-positive cells is from about 30% to about 50%, which is a value significantly higher than the value in the WT that is a comparative example. In addition, in Part E of FIG. 6-4, although the ratio of the frame-shift GFP-positive cells in the quadruple variant D195K/D208R/V232A/S188R is about 30% that is minimum, it is clearly shown that the value is significantly higher than that of the WT. It is shown that a quadruple variant satisfying such a condition that the mutations of D195K/D208R/V232A are included is particularly useful because a high value is stably obtained for the ratio of the frame-shift GFP-positive cells as described above. Further, in each of quadruple variants D195K/D208R/V232A/I23K, D195K/D208R/V232A/K80R, D195K/D208R/V232A/Q93M, and D195K/D208R/V232A/I123H, the ratio of the frame-shift GFP-positive cells is a value comparable to or higher than that in the D195K/D208R/V232A triple variant. Accordingly, it is shown that those variants are particularly useful quadruple variants. In addition, it is strongly estimated from the foregoing that not only quadruple variants each including D195K/D208R/V232A but also m-fold variants ("m" represents a natural number of 4 or more) each including at least the mutations of D195K/D208R/V232A are useful.

In Part E of FIG. 6-4, in the quadruple variant of the combination of D195K/D208R/V232A and S188R, although an extremely high value of about 10 times as high as that of the WT is obtained, the value is lower than those of the other quadruple variants. Accordingly, it is estimated that a high value is obtained in a variant that is free of the mutation of S188R out of quadruple variants, quintuple variants, sextuple variants, septuple variants, ···, and m-fold variants ("m" represents a natural number of 4 or more) each including the mutations of D195K/D208R/V232A.

In addition to the fact that the double variants, triple variants, and quadruple variants shown in Parts C to E of FIG. 6-4 are suitable, it is also estimated that high values are obtained in triple variants, quadruple variants, quintuple variants, sextuple variants, septuple variants, octuple variants, ···, and m-fold variants ("m" represents a natural number of 3 or more) further having amino acid substitutions from these variants. With regard to each of those variants, when its sequence identity with the amino acid sequence as set forth in SEQ ID NO: 1 becomes extremely low, its cleavage activity against a target site may be lost. However, it is estimated that in the case of a variant whose sequence identity is high to some extent, the ratio of the frame-shift GFP-positive cells is high and hence high cleavage activity is obtained as in those variants.

In the example of Part E of FIG. 6-4, with regard to each of: a double variant having at least a substitution to histidine (H) at amino acid position 188 in the amino acid sequence as set forth in SEQ ID NO: 1; and a double variant having at least a substitution to lysine (K) at amino acid position 195 in the amino acid sequence as set forth in SEQ ID NO: 1, a variant having cleavage activity against a target site and having a sequence identity of, for example, 90% or more, preferably 95% or more, more preferably 98% or more with the amino acid sequence as set forth in SEQ ID NO: 1 is suitable.

Similarly, with regard to each of the triple variants shown in Part D of FIG. 6-4 and the quadruple variants shown in Part E of FIG. 6-4, a variant having cleavage activity against a target site and having a sequence identity of 90% or more, preferably 95% or more, more preferably 98% or more with the amino acid sequence as set forth in SEQ ID NO: 1 is suitable.

In addition, as described above, even higher genome editing activity is stably obtained in a variant that is free of the mutation of D208R out of double variants, triple variants, quadruple variants, quintuple variants, sextuple variants, ···, and m-fold variants ("m" represents a natural number of 2 or more) each including the mutation of S188H. Accordingly, in each of those variants, its cleavage activity against a target site becomes higher even in a range where its sequence identity with the amino acid sequence as set forth in SEQ ID NO: 1 is lower. A variant having a sequence identity lower than those of the above-mentioned variants, for example, a variant having a sequence identity of, for example, 88% or more with the amino acid sequence as set forth in SEQ ID NO: 1 and having cleavage activity against a target site may also be suitable. Similarly, it is estimated that a high value is obtained in a variant that is free of the mutation of S188R out of quadruple variants, quintuple variants, sextuple variants, septuple variants, ···, and m-fold variants ("m" represents a natural number of 4 or more) each including the mutations of D195K/D208R/V232A.

### <Optimization of sgRNA for AsCas12f>

Part A of FIG. 7-1 is an explanatory view of the sequences of sgRNA variants (sgRNA_ΔS3-5_v1 to sgRNA_ΔS3-5_v7), in which Stems 3 and 4 are eliminated, and the lengths and sequences of linker regions are modified in order to maintain the original architectures of the remaining regions. The linker regions are surrounded by a red dotted frame.

Part B of FIG. 7-1 Is an explanatory graph showing the genome editing activities of the sgRNA variants determined by flow cytometry. Part C of FIG. 7-1 is a graph showing the expression of sgRNA quantified by a qPCR for a common sequence site (n=3, mean±SD). Part D of FIG. 7-1 is a graph showing the temporal course of GFP deletion caused by various Cas enzymes in HEK293T cells each expressing d2EGFP (n=3, mean±SD). Each sgRNA was introduced by single-copy lentivirus infection. The GFP deletion by AsCas12f-HKRA with sgRNA _ΔS3-5_v7 is slightly lower than deletion by SpCas9, but its deletion rate is faster than deletion rates observed in AsCas12a, enAsCas12a, and Ultra. In Part D of FIG. 7-1, (a) shows results in WT+ΔS5, (b) shows results in WT+ΔS3-5_v7, (c) shows results in HKRA+ΔS5, (d) shows results in WHKRA+ΔS3-5_v7, (e) shows results in AsCas12a, (f) shows results in enAsCas12a, (g) shows results in Ultra, and (h) shows results in SpCas9.

Part E of FIG. 7-2 is a schematic explanatory view of wild-type sgRNA, and Part F of FIG. 7-2 is a schematic explanatory view of sgRNA_ΔS3-5_v7. Disordered regions are illustrated in grey. PK1, PK2, Stem 1, and Stem 2 form extensive interactions with an AsCas12 protein. However, Stem 3 and Stem 4 form substantially no interactions with the protein, and Stem 5 is disordered. Accordingly, sgRNA_ΔS3-5_v7 is designed by eliminating Stems 3 to 5, and the eliminated region is illustrated as being surrounded by a dotted line. The remaining regions each maintain an original architecture.

Part G of FIG. 7-3 is an illustration of the structure of a wild-type sgRNA scaffold, and Part H of FIG. 7-3 is an illustration of the structure of a sgRNA_ΔS3-5_v7 scaffold. Disordered regions are indicated by dotted lines. Part I of FIG. 7-4 is an enlarged view of the PK1 region of the wild-type sgRNA, and Part J of FIG. 7-4 is an enlarged view of the PK1 region of sgRNA_ΔS3-5_v7. Part K of FIG. 7-4 is an illustration of the PK2 region of the wild-type sgRNA, and Part L of FIG. 7-4 is an enlarged view of the Stem 3 region of sgRNA_ΔS3-5_v7.

In the structure of this embodiment, Stem 5 is completely disordered. In addition, it is illustrated that while PK1, PK2, Stem 1, and Stem 2 interact extensively with an AsCas12f protein, Stem 3 and Stem 4 are exposed to a solvent and substantially free from interacting with the protein.

Accordingly, the sgRNA was further engineered by performing the truncation of those domains as illustrated in Part A of FIG. 7-1. The seven sgRNA variants (sgRNA_ΔS3-5_v1 to sgRNA _ΔS3-5_v7) were designed to eliminate Stem 3 and Stem 4, and the remaining regions were each caused to maintain an original structure. As a result, as shown in Part B of FIG. 7-1, all the sgRNA variants each showed an increase in genome editing activity as compared to sgRNA_ΔS5. In particular, sgRNA_ΔS3-5_v7 was most effective.

As shown in Part C of FIG. 7-1, the expression level of sgRNA_ΔS3-5_v7 in HEK293 cells was 4.5 times as high as the expression level of sgRNA_ΔS5. The foregoing shows that the shortening of the sgRNA increases its expression level, and hence enhances its genome editing activity. In order to further clarify the effects of sgRNA modification, sgRNA_ΔS5 and sgRNA_ΔS3-5_v7 each targeting a GFP were introduced by infection with one copy of lentivirus, and were each evaluated for the deletion rate of the GFP. As shown in Part F of FIG. 5-2 and Part D of FIG. 7-1, sgRNA_ΔS3-5_v7 significantly improved the GFP deletion efficiency of each of both AsCas12f-WT and AsCas12f-HKRA as compared to sgRNA_ΔS5. Accordingly, it was suggested that sgRNA_ΔS3-5_v7 was effective in situations where an expression level was limited such as in vivo gene delivery.

### <Structures of sgRNA-optimized AsCas12f Variants measured with Cryo-electron Microscope>

Part A of FIG. 8-1 is an illustration of the structural models of AsCas12f-sgRNA-target DNA (left), AsCas12f-YHAM-sgRNA_ΔS3-5_v7-target DNA (center), and AsCas12f-HKRA-sgRNA_ΔS3-5_v7-target DNA (right) complexes. Mutated residues are illustrated as CPK models.

Part B of FIG. 8-2 is an illustration of the dimer interface of AsCas 12f-YHAM, Part C of FIG. 8-2 is an illustration of the target DNA recognition of AsCas12f-YHAM, and Part D of FIG. 8-2 is an illustration of the hydrophobic interaction of AsCas12f-YHAM. Mutated residues are highlighted in red and are represented by dark-colored portions in the figure. Part E of FIG. 8-2 is an illustration of the recognition of the guide RNA-target DNA heteroduplex of AsCas12f-HKRA, Part F of FIG. 8-2 is an illustration of the recognition of target DNA, and Part G of FIG. 8-2 is an illustration of the recognition of a sgRNA scaffold. Mutated residues are highlighted in red and are represented by dark-colored portions in the figure.

As shown in Part A of FIG. 8-1, Parts E to L of FIG. 2-3 to FIG. 2-6, and FIG. 9 and FIG. 10 that are explanatory tables of the results of data collection, processing, model refinement, and validation, in order to obtain a mechanistic insight into the enhancement of DNA cleavage activity exhibited by an AsCas12f variant, the structures of the AsCas12f-YHAM-sgRNA_ΔS3-5_v7-target DNA complex and the AsCas12f-HKRA-sgRNA_ΔS3-5_v7-target DNA complex were measured with a cryo-electron microscope at an overall resolution of 2.9 Å in both cases. As a result, it was clarified that mutations introduced by DMS experiments did not have any major influences on the overall structures of the complexes because the overall structures of the AsCas12f-YHAM variant and the AsCas12f-HKRA variant were similar to the structure of wild-type AsCas12f. In Part A of FIG. 8-1, the introduced mutations are shown in CPK display (displayed by color coding in accordance with atom species).

Explanatory views of original wild-type sgRNA are illustrated in Parts E, G, I, and K of FIG. 7-2 to FIG. 7-4, and explanatory views of sgRNA_ΔS3-5_v7, which is a modified sgRNA variant, are illustrated in Parts F, H, J, and L of FIG. 7-2 to FIG. 7-4 corresponding thereto, respectively. As illustrated in the figures, sgRNA_ΔS3-5_v7 includes a 20-nt guide segment (from G1 to C20) and a 96-nt sgRNA scaffold (from A(-96) to C(-1)). sgRNA_ΔS3-5_v7 interacts extensively with AsCas12f as follows: while Stem 1, 2, and PK1 regions are maintained, Stem 3, 4, and 5 regions are deleted.

As illustrated in Parts I and J of FIG. 7-4, in particular, C(-6) corresponding to inverted C(-103) of the wild-type sgRNA forms a base pair with G(-83), and connects PK1 and Stem 1 to form a continuous helix. As illustrated in Parts K and L of FIG. 7-4, while a Stem 3 region corresponding to a wild-type PK2 region maintains G(-24):C(-8) and G(-23):C(-9) base pairs (G(-122):C(-88) and G(-121):C(-87) in a wild type), G(-12) is inverted and stacked with Trp17. The foregoing is similar to an interaction between G(-90) and Trp17 in the wild type. Unexpectedly, as illustrated in Part L of FIG. 7-4, A(-10) forms a non-canonical base pair with U(-22), and connects base pairs from G(-24):C(-8) to G(-23):C(-9) and from C(-21):G(-14) to C(-20):G(-15) in Stem 3. The foregoing is a description of the fact that sgRNA_ΔS3-5_v7 is an optimal sgRNA variant.

Stem 1 to Stem 5 are each a stem loop, and create an optimal sgRNA variant by adjusting the sequence and length of a linker region connecting deleted portions. Thus, it was recognized that genome editing activity was improved by deleting part of sgRNA in different Cas enzymes. A possible reason for the improvement in genome editing activity is as follows: the shortening of the length of the sgRNA improves the transcription level of the sgRNA in introduced mammalian cells, and hence makes it easier for the sgRNA to form a complex with Cas. In particular, it was shown from the structure of AsCas12f that Stem 4 and Stem 5 formed substantially no interactions with AsCas12f, and hence a short sgRNA variant from which these portions were deleted was created.

Parts B to G of FIG. 8-2 are explanatory views for illustrating introduced mutations. An F48Y mutation indicated by Y48.1 in Part B of FIG. 8-2 is present at a dimer interface, and turns into Y to form a new interaction with another molecule, thereby strengthening the assembly of a dimer. S188H indicated by H188.1 in Part C of FIG. 8-2 forms a new hydrophobic interaction with a nucleic acid. E316M, which is indicated by each of M316.1 in AsCas12f.1 and M316.2 in AsCas12f.2 in Part D of FIG. 8-2, contributes to stability as a protein through the turning of E into M because its surroundings are surrounded by hydrophobic residues. I123H/D195K indicated by H123.1 and K195.1 in Part E of FIG. 8-2 form new interactions with RNA. 208R, which is indicated by R208.1 in Part F of FIG. 8-2 and indicated by R208.2 in Part G of FIG. 8-2, forms new interactions with DNA/RNA. The genome editing activity of the variant is increased by the formation of those new interactions and the increase in stability.

Specifically, as illustrated in Part B of FIG. 8-2, in an AsCas12f-YHAM structure, Tyr48.1 (F48Y.1, described as Y48.1 in the figure) positioned at the dimer interface may promote more stable dimerization by forming a hydrogen bond with the main chain of G57.2 in addition to a hydrophobic interaction observed in the wild-type AsCas12f. As illustrated in Part C of FIG. 8-2, a side chain of His188.1 (S188H.1, described as H188.1 in the figure) is stabilized by Ile2, and interacts with a backbone phosphate group between dT19 and dC18. Thus, it is shown that an S188H mutation promotes the unwinding of the target DNA. As illustrated in Part D of FIG. 8-2, Met316.1 and Met316.2 (E316M.1 and E316M.2, described as M316.1 and M316.2 in the figure) may form further hydrophobic interactions with Thr239 and Ala241 to enhance overall stability.

As illustrated in Part E of FIG. 8-2, in an AsCas12f-HKRA structure, His123.1 (described as H123.1 in the figure) and Lys195.1 (described as K195.1 in the figure) provide hydrophobic and electrostatic interactions with the ribose portion of A5, and the backbone phosphate groups of A3 and A5, respectively to stabilize a guide RNA-target DNA heteroduplex. As illustrated in Part F of FIG. 8-2, Arg208.1 (described as R208.1 in the figure) forms a hydrogen bond and a hydrophobic interaction with dT19 and Ile2, respectively. The foregoing is similar to His188.1 of AsCas12f-YHAM, and initial heteroduplex formation is thus promoted. Further, as illustrated in Part G of FIG. 8-2, Arg208.2 of another subunit (described as R208.2 in the figure) also forms an electrostatic interaction with the phosphate backbone of G(-43) in the sgRNA scaffold to stabilize a complex structure.

No clear finding regarding the contribution of a V232A mutation to an improvement in DNA cleavage activity of each of both the AsCas12f-YHAM and AsCas12f-HKRA variants was obtained in this structural analysis, though the V232A mutation was present in each of both the variants. Those findings suggest that an engineering approach based on DMS has a great potential to produce a high-activity variant that cannot be predicted only from structural information.

### <Characterization of AsCas12f Variants in Human Cells>

Part A of FIG.11-1 is an explanatory view of the measurement of PAM specificity (specificity), and Part B thereof is a graph showing the results thereof. It is shown that a wild type (WT) causes substantially no genome editing at any PAM, but the two variants according to this embodiment each cause genome editing at a TTR PAM. The axis of ordinate of Part B of FIG. 11-1 indicates an editing frequency (%), and bar graphs show values in the wild type (WT), AsCas12f-YHAM, and AsCas 12f-HKRA from the left in the figure, respectively. Parts C to E of FIG. 11-2 are graphs showing genome editing activities with various Cas enzymes. In the figure, SpCas9 represents the most commonly used high-activity Cas9, AsCas12a represents the most commonly used high-activity Cas12a (Cpf1), and Ultra and enAsCas12a represent modified versions of AsCas12a, respectively.

It was found from those results that the AsCas12f variants according to this embodiment (AsCas12f-YHAM and AsCas12f-HKRA) each exhibited activity higher than, or activity equivalent to or higher than, that of each of those commonly used Cas enzymes depending on target genes.

Part A of FIG. 12-1 is a graph showing the comparison results of target editing efficiencies in AsCas12f-YHAM and AsCas12f-HKRA. As shown in Part A of FIG. 11-1 and Part A of FIG. 12-1, in order to evaluate the PAM specificities of enAsCas12f variants for a wide range of targets, a self-targeting library having both sgRNA and a target sequence thereof was developed, and indel formations induced by wild-type AsCas12f (described as AsCas12f for simplification of description), AsCas12f-YHAM, and AsCas12f-HKRA were measured for 750 different spacer sequences each having 16 NTTN PAMs. As illustrated in Part B of FIG. 12-1, as a result of deep sequencing analysis, the three enzymes induced indels at an NTTR PAM, but did not induce indels at an NTTY (Y represents T or C) PAM, and hence it was shown that those enzymes each recognized an NTTR sequence as a PAM. That is, it can be said that AsCas12f recognizes TTR (R represents A or G) as a PAM.

As illustrated in Part B of FIG. 12-1, it was clarified from a structure observed with a cryo-electron microscope that the nucleobases of dT(-3 *) and dT(-2*) of a PAM duplex formed hydrophobic interactions with Tyr76.1, and N6 of dA(-1*) formed a hydrogen bond with His72.1. A clear explanation for the preference (selectivity) of the NTTR PAM was obtained because it was suggested by modeling that O6 of dG(-1 *) formed a hydrogen bond with His72.1. As shown in Part B of FIG. 11-1, at the NTTR PAM, AsCas12f induced an average of 3.0% of indels, but AsCas12f-YHAM and AsCas12f-HKRA induced averages of 40.8% and 44.7% of indels, respectively.

It became clear from those results that the enAsCas12f variants each exhibited much higher genome editing activity than that of AsCas12f in each of various target sequences each using the NTTR PAM. Next, the genome editing efficiency of each of AsCas12f and the enAsCas12f variants at each of five target sites each using the NTTG PAM was compared to the genome editing efficiency of each of Cas12a and Cas12a variants (UltraCas12a and enAsCas12a) by using HEK293T cells.

In Part C of FIG. 11-2, bar graphs for each item, such as VEGFA, HEXA, ···, show indel frequencies (%) in AsCas12f, AsCas12f-YHAM, AsCas12f-HKRA, AsCas12a, UltraCas12a, and enAsCas12a from the left in the figure, respectively. In those five sites, AsCas12f, AsCas12f-YHAM, AsCas12f-HKRA, AsCas12a, UltraCas12a, and enAsCas12a generated indels at average frequencies of 8.5%, 22.3%, 14.3%, 9.0%, 11.0%, and 11.1%, respectively. Those results show the following: the enAsCas12f variants can each induce indels with efficiency equivalent to or greater than those of Cas12a and the Cas12a variants; in contrast, no indel induction with such efficiency occurs in AsCas12f. In addition, in Parts D and E of FIG. 11-2, bar graphs for each item show measurement results in AsCas12f, AsCas12f-YHAM, AsCas12f-HKRA, and SpCas9 from the left in the figure, respectively.

As illustrated in Part D of FIG.11-2, the genome editing efficiency of AsCas12f was compared to the genome editing efficiency of SpCas9 at each of eight target sites in the HEK293T cells. Those eight target sites each have the same spacer sequence as that of a 5'-TTTG PAM for AsCas12f or that of an NGG-3' PAM for SpCas9. As illustrated in the figure, AsCas12f, AsCas12f-YHAM, AsCas12f-HKRA, and SpCas9 generated indels at average frequencies of 1.7%, 14.8%, 16.9%, and 22.9%, respectively at the eight target sites. Further, the formation of indels in three therapeutic targets, namely, PCSK9 and ANGPTL3 for atherosclerosis, and TTR for transthyretin amyloidosis was also measured in the HEK293T cells.

As shown in Part E of FIG. 11-2, higher indel frequencies were observed in AsCas12f-YHAM and AsCas12f-HKRA than in SpCas9 at two of the three target sites. As shown in Parts C and D of FIG. 12-2, and Part E of FIG.12-3, similar results were obtained at other target sites and in other human cell lines, such as Huh-7 hepatoma cells and HT-1080 sarcoma cells. Thus, AsCas12f-YHAM and AsCas12f-HKRA were shown to efficiently induce indels regardless of target sites and cell lines.

Next, mismatch tolerance was examined in order to investigate the specificity of each of the enAsCas12f variants. Part A of FIG. 13-1 is a graph showing the influence of a mismatch on EGFP activation mediated by AsCas12f-YHAM and AsCas12f-HKRA in a Split-GFP-reframing assay using a TTR sequence (n=3). The symbol "-" at the left end of the graph shows data when normal guide RNA perfectly complementary to target DNA is used. The terms "M01", "M02", ··· each mean a position where the mismatch is introduced between the guide RNA and the target DNA, that is, a position where an incorrect guide sequence is used so as not to form a base pair. Accordingly, the term "M01" means that the mismatch is present at the first position of the guide RNA, and the term "M01/02" means a double-mismatch guide RNA in which mismatches are present at the first and second positions of the guide RNA. In Part A of FIG. 13-1, its axis of ordinate indicates indel efficiency (%), and in the respective bar graphs for M01, M02, M03, ···, a bar graph on the left side in the figure shows a value in AsCas12f-YHAM, and a bar graph on the right side therein shows a value in AsCas 12f-HKRA.

As shown in Part A of FIG. 13-1, as observed in Cas12a and the other Cas12fs, broad tolerance to single mismatches was shown in both of the enAsCas12f variants except for a PAM distal region (positions 16 to 20), but tolerance to double mismatches was negligibly small. Further, the genome-wide specificity of each of AsCast2f-HKRA and SpCas9 was examined by using genome-wide, unbiased identification of double-stranded breaks enabled by sequencing (GUIDE-seq) at nine target sites. Part B of FIG.13-1 is an explanatory view of the numbers of off-target sites for AsCas12f-HKRA and SpCas9 detected by GUIDE-seq at the same spacer site as in Part D of FIG. 11-2, and Part C-1 of FIG. 13-2, Part C-2 of FIG. 13-3, and Part C-3 of FIG. 13-4 are each an explanatory view of off-target sites identified by the GUIDE-seq.

As shown in Part B of FIG. 13-1, Part C-1 of FIG. 13-2, Part C-2 of FIG. 13-3, and Part C-3 of FIG. 13-4, AsCas12f-HKRA and SpCas9 had about the same number of off-target sites, and AsCas12f-HKRA tolerated mismatches in a 5-nt PAM distal region. Those results were consistent with those of the mismatch experiment. Those results show that the enAsCas12f variants each having sgRNA_ΔS3-5_v7 each exhibit genome editing activity and specificity equivalent to or greater than those of SpCas9 and Cas12a despite their extremely compact sizes.

### <Therapeutic Potential of Genome Editing mediated by AsCas12f>

FIG. 14-1 and FIG. 14-2 are explanatory views of the production of muscular dystrophy model induced pluripotent stem cells (iPSCs) and their differentiation into cardiomyocytes (iPSC-CMs). Part A of FIG.14-1 is an explanatory view of the recognition of the complete deletion of a DMD exon by a PCR, and Part B of FIG. 14-1 is an explanatory view of the genomic sequence of a DMD exon 44-deleted iPS cell line. Part C of FIG. 14-2 is a graph showing the flow cytometry analysis of α-actinin-mCherry on day 14 of myocardial differentiation. It is shown that all DMD exon 44-deleted lines efficiently differentiated into cardiomyocytes without purification. Part D of FIG. 14-2 is a view for illustrating the Western blot analysis of AR and DMD iPSC cardiomyocytes (#2 to 16, 33, 66, and 84). Arrow lines in the figure indicate the immunoreactive bands of dystrophin, and asterisks indicate non-specific bands. Part E of FIG. 14-3 is a graph showing luciferase activity comparison between dAsCas12f WT and dAsCas12f-HKRA (n=3, mean±SD). Part F of FIG. 14-3 is a schematic explanatory view of the plasmid construct of dAsCas12f in which VP64 or VPR is bound to different sites. A gRNA targeting HEXA and a plasmid were transduced into Huh-7 cells each stably expressing a luciferase under the control of a minimal CMV promoter having a HEXA gRNA binding site. Part G of FIG. 14-3 is a graph showing an increase in luciferase activity in cells having introduced thereinto a plasmid vector (n=3, mean±SD).

As shown in Parts A to D of FIG. 14-1 and FIG. 14-2, in order to evaluate a possibility of application to therapy, induced pluripotent stem cells (iPSCs) from which Duchenne muscular dystrophy (DMD) exon 44 (hDMDΔEx44) serving as a major mutation of DMD was deleted were produced, and were differentiated into cardiomyocytes (iPSC-CMs). The deficiency of dystrophin causes myopathy and cardiomyopathy, and exon 45 skipping that restores a DMD protein has been studied.

Parts A to F of FIG. 15-1 and Parts G to J of FIG. 15-2 are explanatory views and graphs of treatment and knock-in with variants. Specifically, Parts A to C of FIG. 15-1 are explanatory views of treatment for dystrophin with the AsCas12f variants (iPSCs). Parts D to F of FIG. 15-1 are explanatory view and graphs of treatment for transthyretin with the AsCas12f variants (administered by using an AAV to mice). In Part E of FIG. 15-1, its axis of ordinate indicates plasma transthyretin (µg/ml), and its axis of abscissa indicates days elapsed (weeks). In addition, (a) shows the value of plasma transthyretin in AsCas12f at 3.0×10¹¹ vg, (b) shows that in AsCas12f at 1.0×10¹² vg, (c) shows that in HKRA at 3.0×10¹¹ vg, and (d) shows that in HKRA at 1.0×10¹² vg. In addition, in Part F of FIG. 15-1, its axis of ordinate indicates an indel frequency (%), and on its axis of abscissa, (a) shows AsCas12f at 3.0×10¹¹ vg, (b) shows AsCas12f at 1.0×10¹² vg, (c) shows HKRA at 3.0×10¹¹ vg, and (d) shows HKRA at 1.0×10¹² vg. Parts G to I of FIG. 15-2 show the knock-in of GFP/FIX at an Alb locus with the AsCas12f variants (mammalian cells). In Part I of FIG. 15-2, its axis of ordinate indicates the ratio (%) of EGFP-positive cells (positive cells), and in Part J thereof, its axis of ordinate "FIX:C (%)" indicates an increase in plasma factor IX activity (FIX:C).

As shown in Part A of FIG. 15-1, AsCas12f-HKRA was found to efficiently reduce the amount of a dystrophin protein as a result of the expression of AsCas12f-HKRA and sgRNA targeting a DMD gene with an all-in-one AAV serotype 6 vector in WT iPSC-CMs. As shown in Parts B and C of FIG. 15-1, further, AsCas12f-HKRA partially restored the dystrophin protein of hDMDΔEx44-iPSC-CMs when paired with sgRNA designed to skip exon 45. The possibility of DMD treatment by AAV gene delivery is recognized from the foregoing.

Next, AsCas12f-HKRA was applied to the genome editing of a mouse liver in order to investigate the therapeutic potential of AsCas12f in vivo. As illustrated in Part D of FIG. 15-1, an AAV vector encoding AsCas12f (or AsCas12f-HKRA) under an HCRhAAT promoter, and sgRNA working under the control of a U6 promoter (promoter-driven sgRNA) targeting a TTR gene for transthyretin amyloidosis were constructed. Liver-tropic AAV serotype 8 was injected at 3×10¹¹ vg or 1×10¹² vg into 7-week-old mice, and the expression levels of plasma transthyretin were evaluated. As shown in Part E of FIG. 15-1, plasma transthyretin levels were not able to be regulated with AsCas12f, but AsCas12f-HKRA reduced the plasma transthyretin levels in a dose-dependent manner. When a target locus was analyzed by target amplicon deep sequencing 8 weeks after AAV-based gene delivery, a high editing rate (66.3%) was achieved by 1×10¹² vg of AsCas12f-HKRA (Part F of FIG. 15-1).

Next, the in vivo knock-in efficiency of an AsCas12f system for inserting an EGFP gene into an mAlb 3'UTR was evaluated. The knock-in of a gene at the Alb locus is a platform studied for ectopically producing therapeutic proteins including coagulation factors and lysosomal enzymes from the liver. When two AAV vectors were prepared, one expressed AsCas12f or AsCas12f-HKRA and sgRNA targeting the mAlb 3'UTR, and the other provided a donor template for knocking in an EGFP into only a DSB through homology-directed repair (HDR) (Part G of FIG. 15-2). Those two AAV vectors were injected intraperitoneally into C57BL/6 wild-type newborn mice, and EGFP expression in their livers 4 weeks after the vector injection was evaluated with an immunofluorescence microscope.

As a result, as shown in Parts H and I of FIG. 15-2, a significant increase in number of EGFP-positive hepatocytes was recognized by the injection of the AAV vector carrying AsCas12f-HKRA. Next, the EGFP gene of a donor vector was replaced with coagulation factor IX (F9) cDNA having a Padua mutation, and the vector was injected into newborn hemophilia B mice (F9-deficient mice). As shown in Part J of FIG. 15-2, the knock-in of an F9 gene at the Alb locus with AsCas12f-HKRA significantly increased plasma coagulation factor IX (FIX) activity beyond a therapeutic range as compared to a case of donor only. However, such increase was not seen in AsCas12f. Those pieces of data show that the AsCas12f variant having optimal sgRNA can be utilized for in vivo gene therapy, for example, for hemophilia.

### <Application of Compact enAsCas12f>

An AsCas12f gene can be packaged into an all-in-one AAV vector together with a plurality of sgRNAs and a large partner gene because its size is small. Accordingly, the gene is applicable to genome editing therapy, which has been impossible with conventional genome editing tools. Parts A to F of FIG. 16-1 are explanatory views and graphs of the knock-in of FIX at an Alb locus (mice) with the AsCas12f variants. Parts G to J of FIG. 16-2 are explanatory views and graphs of gene transcriptional activation (CRISPR activation) (mammalian cells) with the AsCas12f variants. As shown in the figures, a significant increase in transcriptional activity is achieved by simultaneously introducing MS2.

As illustrated in Part A of FIG. 16-1, a single AAV vector encoding AsCas12f (or AsCas12f-HKRA) under a liver-tropic Ttr promoter, a donor sequence, and sgRNA working under the control of a U6 promoter was designed in order to insert F9 cDNA having a Padua mutation into an Alb 3'UTR locus. As shown in Parts B and C of FIG. 16-1, when a single AAV serotype 8 vector was injected into newborn hemophilia B mice, both of plasma FIX activity (FIX:C) and an antigen (FIX:Ag) were significantly increased by an AAV vector carrying AsCas12f-HKRA, but the activity and the antigen were not increased by an AAV vector carrying AsCas12f. As shown in Parts D and E of FIG. 16-1, a clotting time evaluated by an activated partial thromboplastin time (APTT) and the expression level of F9 mRNA evaluated by a quantitative RT-PCR were significantly improved, the improvement being consistent with the foregoing results. As shown in Part F of FIG.16-1, the amylose gel analysis of mRNA PCR fragments revealed that cDNA insertion by HDR occurred, though insertion by non-homologous end joining (NHEJ) was also observed.

Finally, as illustrated in Part G of FIG. 16-2, in order to investigate the utility of enAsCas12f in epigenome editing, a transcriptional activation assay was performed with Huh-7 cells each stably expressing a luciferase, the cells being driven by a minimal CMV promoter having two HEXA gRNA recognition sites. Single guide RNA in which an MS2 aptamer was inserted into a stem loop thereof was produced in order to enhance the transcriptional activity of enAsCas12f. As illustrated in Part G of FIG. 16-2, a plasmid expressing AsCas12f-HKRA whose DNA cleavage activity was eliminated (dead AsCas12f-HKRA, hereinafter referred to as "dAsCas12f-HKRA"), which was joined to VP64 and an MS2 fusion activator (MS2-p65-HSF1), and engineered sgRNA targeting HEXA were genetically introduced into Huh-7 cells.

As shown in Part H of FIG.16-2 and Part E of FIG. 14-3, dAsCas12f-HKRA combined with the sgRNA having inserted thereinto the MS2 aptamer significantly enhanced luciferase expression. As shown in Parts F and G of FIG.14-3, the binding of VP64 at an end of dAsCas12f-HKRA was most effective for transcriptional activation, though direct joining of VPR (VP64, p65, and Rta) was not able to enhance transcription by MS2-p65-HSF1. Further, as shown in Parts I and J of FIG. 16-2, it was recognized that the luciferase expression significantly increased in a dose-dependent manner as a result of the introduction of the Huh-7 cells with a single AAV serotype 6 vector encoding JVP64, MS2-p65-HSF1, and dAsCas12f-HKRA joined to the sgRNA. Those results show that enAsCas12f can be utilized as a transcriptional activation tool.

Part A of FIG. 17 is a pair of explanatory photographs of an example in which an AAV8-type vector expressing a luciferase, the vector being driven by a minimal CMV promoter having two HEXA gRNA recognition sites, and an AAV8-type vector having a structure illustrated in Part I of FIG. 16-2 were simultaneously intravenously administered to wild-type mice. The pair of photographs is obtained by converting a color heat map, in which a count value increases as a color changes from blue (50 counts) to red (200 counts), into grayscale. In Part A of FIG. 17, it is shown that, as compared to four individual mice of AsCas12f WT (left side), in a first mouse from the left of AsCas12f HKRA (right side), a cyclic region shown in dark gray having a count value of from about 50 to about 100, a cyclic region therein shown in light gray having a count value of from about 100 to about 170, and a small region therein shown in slightly dark gray having a count value of from about 170 to about 200 are present, though the regions are shown in grayscale. It is shown that, in a second mouse from the left in the photograph, regions shown in dark gray having a count value of from about 50 to about 100, and regions shown in light gray having a count value of from about 100 to about 170, are scattered. It is shown that, in third and fourth mice from the left in the photograph, a cyclic region shown in dark gray having a count value of from about 50 to about 100, a cyclic region therein shown in light gray having a count value of from about 100 to about 170, and a wide closed region therein shown in slightly dark gray having a count value of from about 170 to about 200 are present.

Part B of FIG. 17 is a graph showing ROI values (axis of ordinate) in the WT and HKRA. In addition, as shown in the graph, luciferase activity increased about 10 times in Cas12f-HKRA as compared to the wild-type AsCas12f. It is shown that the increase is statistically significant because P=0.0474 (p<0.05) as shown in the graph.

In view of the result, it is conceivable that the transcription of endogenous genes can be increased by about 10 times. For example, it is conceivable that an increase in transcription of utrophin that is fetal dystrophin enables the treatment of Duchenne muscular dystrophy.

FIG. 18 shows an explanatory table of nucleic acid sequences used for structural analysis in this embodiment. FIG. 19 shows an explanatory table of nucleic acid sequences used for structural analysis in relation to STAR Methods. FIG. 20-1 to FIG. 20-4 show explanatory tables of primers and constructs used for genome editing in relation to STAR Methods. FIG. 21-1 to FIG. 21-7 show explanatory tables of key resources in STAR Methods. Those are exemplary and do not limit the invention. In addition, in FIG. 19 to FIG. 20-4, 3A and 3B, and 3C to 3F correspond to Parts A and B of FIG. 5-1, and Parts C to F of FIG. 5-2, respectively, 5A and 5B, and 5C to 5E correspond to Parts A and B of FIG. 11-1, and Parts C to E of FIG. 11-2, respectively, and 6A to 6F and 6G to 6J correspond to Parts A to F of FIG. 15-1 and Parts G to J of FIG. 15-2, respectively. In addition, S4A to S4D, S4E and S4F, S4G and S4H, and S4I to S4L correspond to Parts A to D, E and F, G and H, and I to L of FIG. 7-1, respectively.

As described above, compact AsCas12f variants each having improved activity were successfully produced by applying a deep mutational scanning (DMS) technology to a CRISPR-Cas effector. A DMS approach enables the evaluation of the influences of thousands of mutations in a single experiment through the combination of exhaustive protein mutagenesis and functional screening with deep sequencing. One of typical applications of the DMS is the introduction of a library into a yeast surface display system, and the evaluation of the binding affinities of ligands including antibodies and viral glycoproteins. In this embodiment, a yeast screening system was applied to mammalian cell-based screening in order to evaluate genome editing activity. A library covering all 20 single amino acid substitutions at the respective positions of the entire sequence (422 residues) of AsCas12f was constructed, and 200 or more effective mutations were identified at various positions. It is advantageous to adopt a structural perspective in order to efficiently search for effective combinations of those variants. As described in this embodiment, when there is no experimentally determined structure, a structure prediction model such as AlphaFold can be a useful approach for efficiently selecting mutations identified by the DMS.

Next, an example in which an AsCas12f variant is applied to the genome editing of a plant is described. I123Y/D195K/D208R/V232A was used as the AsCas12f variant.

FIG. 22 is an illustration of the overview of the genome editing of *Nicotiana benthamiana* with a viral vector expressing the AsCas12f variant. As illustrated in the figure, in this example, a target gene was knocked out with the viral vector (PVX) expressing the AsCas12f variant through use of a PDS gene as a target site. Genome editing efficiency comparison was performed for an inoculated leaf (IL) and upper leaves thereof illustrated in FIG. 22 through the infection of the leaves of *Nicotiana benthamiana* with the viral vector by an Agro-infection method for each of SpCas9 and the AsCas12f variant. When the PDS gene was knocked out in all alleles by performing such comparison, cells were whitened, and hence genome-edited cells were visualized. In the figure, IL represents an inoculated leaf, and upper leaves thereof are illustrated as L1, L2, L3, L4, ··· in order from the one closer to the inoculated leaf IL.

The results of the visualization of the genome-edited cells as described above are shown in FIG. 23(a) and FIG. 23(b). FIG. 23(a) shows results by the PVX-AsCas12f variant, and FIG. 23(b) shows results by PVX-SpCas9. As shown in FIG. 23(a), it is clear that for the upper leaf L2, the cells of the leaf are whitened in the PVX-AsCas12f variant. Meanwhile, as shown in FIG. 23(b), substantially no whitening was observed with a naked eye in PVX-SpCas9. In addition, in the PVX-AsCas12f variant, whitening is clearly observed also in the leaves L3 to L7, though the whitening is not as much as that in the leave L2. In addition, the following tendency is observed: whitening appears more strongly as its position is closer to the inoculated leaf IL. Further, also in the leaf L8, whitening is observed at a tip of the leaf. Meanwhile, in PVX-SpCas9, no clear whitening was observed in any of the leaves L2 to L8.

FIG. 24(a) shows NGS analysis results in the case where the PVX-AsCas12f variant and PVX-SpCas9 are each used. As shown in the graph, in the results of NGS analysis using the PVX-AsCas12f variant, a mutation rate is about 70% in each of the inoculated leaf IL and the upper leaf L2, and it is shown that the mutation rate in the upper leaf L2 is slightly higher than that in the inoculated leaf IL. Meanwhile, in the results of NGS analysis using PVX-SpCas9, although the mutation rate showed a value as high as about 80% in the inoculated leaf IL, the mutation rate was 2% in the upper leaf L2. Accordingly, it is shown that the mutation rate is extremely low.

FIG. 24(b) is an illustration of CAPS analysis results in the case where the PVX-AsCas12f variant and PVX-SpCas9 are each used. As illustrated in the figure, in the case where the PVX-AsCas12f variant was used, a black or grey line appeared in a line indicated by a black triangle in the figure in each of the inoculated leaf IL and the upper leaves L2 to L6. Accordingly, it is shown that a mutation is present. Meanwhile, in the case where PVX-SpCas9 was used, although a black line appeared in the inoculated leaf IL, the appearance of a line was not observed in each of the upper leaves L2 to L6. Accordingly, it is shown that there is no mutation.

Thus, it is shown that the use of the AsCas12f variant efficiently causes genome editing in the plant inoculated with the viral vector. In addition, it was shown that the genome editing efficiency was significantly higher also in comparison with the case where PVX-SpCas9 was used. Next, the results of a test using the AsCas12f variant for the genome editing of tomato are described. In the example of FIG. 22 to FIGS. 24, *Nicotiana benthamiana* was used, but in the example of FIG. 25(a) and FIG. 25(b) described below, a similar experiment was performed by using tomato. In this example, as in the example in *Nicotiana benthamiana* of FIG. 22, the genome editing of tomato was performed with the viral vector expressing the AsCas12f variant, and a target gene was knocked out with the viral vector (PVX) expressing the AsCas12f variant through use of the PDS gene as a target site.

FIG. 25(a) is an illustration of the overview of the genome editing of tomato with the viral vector expressing the AsCas12f variant, and FIG. 25(b) is an illustration of the results of CAPS analysis using the PVX-AsCas12f variant. As illustrated in the figure, when the PVX-AsCas12f variant was used, grey lines appeared in a line indicated by a black triangle in the figure in the inoculated leaf IL. Accordingly, it is shown that a mutation is present. In upper leaves L1 and L2, grey lines appeared, though the liners were thinner than those in the inoculated leaf IL. Accordingly, it is shown that mutations are introduced.

In addition, there is no major difference in line density between the upper leaves L1 and L2. Accordingly, genome editing efficiency in the upper leaf L1 and that in the upper leaf L2 were at the same level, and hence a phenomenon in which the genome editing efficiency sharply reduced was not observed. In addition, as in the example in *Nicotiana benthamiana* shown in FIGS. 24, genome editing efficiency that is not much different from that in the upper leaf L1 or L2 may be obtained also in each of leaves further upper than the upper leaf L2.

As described above, AsCas12f variants are useful also for improving genome editing efficiency in plant cells. In addition, it is found from the foregoing that the AsCas12f variants not only improve genome editing efficiency in mammalian cells but also are applicable to an improvement in genome editing efficiency for any animal or plant (or organism) in which cleavage activity can be obtained.

The present invention discloses the following aspects of the invention.

A first aspect is a protein including a substitution to histidine at amino acid position 188 in an amino acid sequence as set forth in SEQ ID NO: 1, wherein the protein further includes one substitution selected from: a substitution to tyrosine at amino acid position 2; a substitution to tyrosine at amino acid position 70; a substitution to arginine at amino acid position 80; a substitution to threonine at amino acid position 105; a substitution to histidine at amino acid position 123; a substitution to lysine at amino acid position 195; a substitution to arginine at amino acid position 208; a substitution to alanine at amino acid position 232; a substitution to methionine at amino acid position 246; a substitution to methionine at amino acid position 316; and a substitution to isoleucine at amino acid position 337, and wherein the protein has a sequence identity of 90% or more with the amino acid sequence as set forth in SEQ ID NO: 1, and has cleavage activity against a target site of DNA.

In the first aspect, the protein is more preferably a double variant including the substitution to histidine at amino acid position 188 and one substitution selected as described above.

A second aspect is the protein according to the first aspect, wherein the protein is free from being substituted at amino acid position 208.

A third aspect is a protein including a substitution to histidine at amino acid position 188 and a substitution to alanine at amino acid position 232 in an amino acid sequence as set forth in SEQ ID NO: 1, wherein the protein further includes one substitution selected from: a substitution to histidine at amino acid position 123; a substitution to methionine at amino acid position 246; a substitution to methionine at amino acid position 316; and a substitution to isoleucine at amino acid position 337, and wherein the protein has a sequence identity of 90% or more with the amino acid sequence as set forth in SEQ ID NO: 1, and has cleavage activity against a target site of DNA.

In the third aspect, the protein is more preferably a triple variant including the substitution to histidine at amino acid position 188, the substitution to alanine at amino acid position 232, and one substitution selected as described above.

A fourth aspect is a protein including a substitution to histidine at amino acid position 188, a substitution to alanine at amino acid position 232, and a substitution to methionine at amino acid position 316 in an amino acid sequence as set forth in SEQ ID NO: 1, wherein the protein further includes one substitution selected from: a substitution to tyrosine at amino acid position 2; a substitution to lysine at amino acid position 23; a substitution to tyrosine at amino acid position 48; a substitution to tyrosine at amino acid position 70; a substitution to arginine at amino acid position 80; a substitution to methionine at amino acid position 93; a substitution to threonine at amino acid position 105; a substitution to histidine at amino acid position 123; a substitution to arginine at amino acid position 208; a substitution to methionine at amino acid position 246; a substitution to asparagine at amino acid position 262; a substitution to threonine at amino acid position 354; a substitution to glutamine at amino acid position 364; and a substitution to asparagine at amino acid position 388, and wherein the protein has a sequence identity of 90% or more with the amino acid sequence as set forth in SEQ ID NO: 1, and has cleavage activity against a target site of DNA.

In the fourth aspect, the protein is more preferably a quadruple variant including the substitution to histidine at amino acid position 188, the substitution to alanine at amino acid position 232, the substitution to methionine at amino acid position 316, and one substitution selected as described above.

A fifth aspect is a protein including a substitution to lysine at amino acid position 195 in an amino acid sequence as set forth in SEQ ID NO: 1, wherein the protein further includes one substitution selected from: a substitution to tyrosine at amino acid position 2; a substitution to tyrosine at amino acid position 70; a substitution to arginine at amino acid position 80; a substitution to threonine at amino acid position 105; a substitution to histidine at amino acid position 123; a substitution to arginine at amino acid position 208; a substitution to alanine at amino acid position 232; a substitution to methionine at amino acid position 246; a substitution to methionine at amino acid position 316; and a substitution to isoleucine at amino acid position 337, and wherein the protein has a sequence identity of 90% or more with the amino acid sequence as set forth in SEQ ID NO: 1, and has cleavage activity against a target site of DNA.

In the fifth aspect, the protein is more preferably a double variant including the substitution to lysine at amino acid position 195 and one substitution selected as described above.

A sixth aspect is a protein including a substitution to lysine at amino acid position 195 and a substitution to alanine at amino acid position 232 in an amino acid sequence as set forth in SEQ ID NO: 1, wherein the protein further includes one substitution selected from: a substitution to tyrosine at amino acid position 70; a substitution to arginine at amino acid position 208; and a substitution to methionine at amino acid position 316, and wherein the protein has a sequence identity of 90% or more with the amino acid sequence as set forth in SEQ ID NO: 1, and has cleavage activity against a target site of DNA.

In the sixth aspect, the protein is more preferably a triple variant including the substitution to lysine at amino acid position 195, the substitution to alanine at amino acid position 232, and one substitution selected as described above.

A seventh aspect is a protein including a substitution to lysine at amino acid position 195, a substitution to arginine at amino acid position 208, and a substitution to alanine at amino acid position 232 in an amino acid sequence as set forth in SEQ ID NO: 1, wherein the protein further includes one substitution selected from: a substitution to tyrosine at amino acid position 2; a substitution to lysine at amino acid position 23; a substitution to tyrosine at amino acid position 48; a substitution to tyrosine at amino acid position 70; a substitution to arginine at amino acid position 80; a substitution to methionine at amino acid position 93; a substitution to threonine at amino acid position 105; a substitution to histidine at amino acid position 123; a substitution to arginine at amino acid position 188; a substitution to methionine at amino acid position 246; a substitution to asparagine at amino acid position 262; a substitution to threonine at amino acid position 354; a substitution to glutamine at amino acid position 364; and a substitution to asparagine at amino acid position 388, and wherein the protein has a sequence identity of 90% or more with the amino acid sequence as set forth in SEQ ID NO: 1, and has cleavage activity against a target site of DNA.

In the seventh aspect, the protein is more preferably a quadruple variant including the substitution to lysine at amino acid position 195, the substitution to arginine at amino acid position 208, the substitution to alanine at amino acid position 232, and one substitution selected as described above.

An eighth aspect is the protein according to any one of the first and third to seventh aspects, wherein the sequence identity is 95% or more.

A ninth aspect is the protein according to any one of the first and third to seventh aspects, wherein the sequence identity is 98% or more.

The amino acid sequence of AsCas12f is as set forth in SEQ ID NO: 1 of the attached sequence listing, and is as described below in single-letter notation.

MIKVYRYEIVKPLDLDWKEFGTILRQLQQETRFALNKATQLAWEWMGFSSDYKDNH
GEYPKSKDILGYTNVHGYAYHTIKTKAYRLNSGNLSQTIKRATDRFKAYQKEILRGD
MSIPSYKRDIPLDLIKENISVNRMNHGDYIASLSLLSNPAKQEMNVKRKISVIIIVRGAG
KTIMDRILSGEYQVSASQIIHDDRKNKWYLNISYDFEPQTRVLDLNKIMGIDLGVAVA
VYMAFQHTPARYKLEGGEIENFRRQVESRRISMLRQGKYAGGARGGHGRDKRIKPIE

QLRDKIANFRDTTNHRYSRYIVDMAIKEGCGTIQMEDLTNIRDIGSRFLQNWTYYDL
QQKIIYKAEEAGIKVIKIDPQYTSQRCSECGNIDSGNRIGQAIFKCRACGYEANADYNA
ARNIAIPNIDKIIAESIK*

## Claims

1. A protein, comprising a substitution to histidine at amino acid position 188 in an amino acid sequence as set forth in SEQ ID NO: 1,
wherein the protein further comprises one substitution selected from:
a substitution to tyrosine at amino acid position 2;
a substitution to tyrosine at amino acid position 70;
a substitution to arginine at amino acid position 80;
a substitution to threonine at amino acid position 105;
a substitution to histidine at amino acid position 123;
a substitution to lysine at amino acid position 195;
a substitution to arginine at amino acid position 208;
a substitution to alanine at amino acid position 232;
a substitution to methionine at amino acid position 246;
a substitution to methionine at amino acid position 316; and
a substitution to isoleucine at amino acid position 337, and
wherein the protein has a sequence identity of 90% or more with the amino acid sequence as set forth in SEQ ID NO: 1, and has cleavage activity against a target site of DNA.

2. The protein according to claim 1, wherein the protein is free from being substituted at amino acid position 208.

3. A protein, comprising a substitution to histidine at amino acid position 188 and a substitution to alanine at amino acid position 232 in an amino acid sequence as set forth in SEQ ID NO: 1,
wherein the protein further comprises one substitution selected from:
a substitution to histidine at amino acid position 123;
a substitution to methionine at amino acid position 246;
a substitution to methionine at amino acid position 316; and
a substitution to isoleucine at amino acid position 337, and
wherein the protein has a sequence identity of 90% or more with the amino acid sequence as set forth in SEQ ID NO: 1, and has cleavage activity against a target site of DNA.

4. A protein, comprising a substitution to histidine at amino acid position 188, a substitution to alanine at amino acid position 232, and a substitution to methionine at amino acid position 316 in an amino acid sequence as set forth in SEQ ID NO: 1,
wherein the protein further comprises one substitution selected from:
a substitution to tyrosine at amino acid position 2;
a substitution to lysine at amino acid position 23;
a substitution to tyrosine at amino acid position 48;
a substitution to tyrosine at amino acid position 70;
a substitution to arginine at amino acid position 80;
a substitution to methionine at amino acid position 93;
a substitution to threonine at amino acid position 105;
a substitution to histidine at amino acid position 123;
a substitution to arginine at amino acid position 208;
a substitution to methionine at amino acid position 246;
a substitution to asparagine at amino acid position 262;
a substitution to threonine at amino acid position 354;
a substitution to glutamine at amino acid position 364; and
a substitution to asparagine at amino acid position 388, and
wherein the protein has a sequence identity of 90% or more with the amino acid sequence as set forth in SEQ ID NO: 1, and has cleavage activity against a target site of DNA.

5. A protein, comprising a substitution to lysine at amino acid position 195 in an amino acid sequence as set forth in SEQ ID NO: 1,
wherein the protein further comprises one substitution selected from:
a substitution to tyrosine at amino acid position 2;
a substitution to tyrosine at amino acid position 70;
a substitution to arginine at amino acid position 80;
a substitution to threonine at amino acid position 105;
a substitution to histidine at amino acid position 123;
a substitution to arginine at amino acid position 208;
a substitution to alanine at amino acid position 232;
a substitution to methionine at amino acid position 246;
a substitution to methionine at amino acid position 316; and
a substitution to isoleucine at amino acid position 337, and
wherein the protein has a sequence identity of 90% or more with the amino acid sequence as set forth in SEQ ID NO: 1, and has cleavage activity against a target site of DNA.

6. A protein, comprising a substitution to lysine at amino acid position 195 and a substitution to alanine at amino acid position 232 in an amino acid sequence as set forth in SEQ ID NO: 1,
wherein the protein further comprises one substitution selected from:
a substitution to tyrosine at amino acid position 70;
a substitution to arginine at amino acid position 208; and
a substitution to methionine at amino acid position 316, and
wherein the protein has a sequence identity of 90% or more with the amino acid sequence as set forth in SEQ ID NO: 1, and has cleavage activity against a target site of DNA.

7. A protein, comprising a substitution to lysine at amino acid position 195, a substitution to arginine at amino acid position 208, and a substitution to alanine at amino acid position 232 in an amino acid sequence as set forth in SEQ ID NO: 1,
wherein the protein further comprises one substitution selected from:
a substitution to tyrosine at amino acid position 2;
a substitution to lysine at amino acid position 23;
a substitution to tyrosine at amino acid position 48;
a substitution to tyrosine at amino acid position 70;
a substitution to arginine at amino acid position 80;
a substitution to methionine at amino acid position 93;
a substitution to threonine at amino acid position 105;
a substitution to histidine at amino acid position 123;
a substitution to arginine at amino acid position 188;
a substitution to methionine at amino acid position 246;
a substitution to asparagine at amino acid position 262;
a substitution to threonine at amino acid position 354;
a substitution to glutamine at amino acid position 364; and
a substitution to asparagine at amino acid position 388, and
wherein the protein has a sequence identity of 90% or more with the amino acid sequence as set forth in SEQ ID NO: 1, and has cleavage activity against a target site of DNA.

8. The protein according to any one of claims 1 and 3 to 7, wherein the sequence identity is 95% or more.

9. The protein according to any one of claims 1 and 3 to 7, wherein the sequence identity is 98% or more.
